(19) Europäisches Patentamt
European Patent Office
Office européen des brevets

(11) Publication number: **0 338 634 B1**

(12) **EUROPEAN PATENT SPECIFICATION**

(45) Date of publication of patent specification: **06.04.94**

(51) Int. Cl.[5]: **C07K 7/08**, C07K 7/10, C12P 21/02, C12N 15/00, A61K 37/02

(21) Application number: **89200967.1**

(22) Date of filing: **17.04.89**

(54) **Viper venom polypeptides and genetic expression.**

(30) Priority: **22.04.88 US 184649**
**22.04.88 US 184653**
**01.02.89 US 303757**

(43) Date of publication of application:
**25.10.89 Bulletin 89/43**

(45) Publication of the grant of the patent:
**06.04.94 Bulletin 94/14**

(84) Designated Contracting States:
**AT BE CH DE ES FR GB GR IT LI LU NL SE**

(56) References cited:
**EP-A- 0 220 957**
**EP-A- 0 317 053**

**THE JOURNAL OF BIOLOGICAL CHEMISTRY, vol. 262, no. 33, 25th November 1987, pages 16157-16163, The American Society for Biochemistry and Molecular Biology, Inc.; T.-F. HUANG et al.: "Trigramin: A low molecular weight peptide inhibiting fibrinogen interaction with platelet receptors expressed on glycoprotein IIB-IIIa complex"**

(73) Proprietor: **MERCK & CO. INC.**
**126, East Lincoln Avenue**
**P.O. Box 2000**
**Rahway New Jersey 07065-0900(US)**

(72) Inventor: **Friedman, Paul A.**
**403 Great Spring Road**
**Rosemont, PA 19010(US)**
Inventor: **Polokoff, Mark A.**
**29 Phyllis Place**
**Randolph New Jersey 07869(US)**
Inventor: **Gould, Robert J.**
**3903 Gatehouse Lane**
**Skippack, PA 19474(US)**
Inventor: **Bencen, Gerard H.**
**340 Arch Street**
**Royersford, PA 19468(US)**
Inventor: **Jacobs, John W.**
**298 W. Court Street**
**Doylestown, PA 18901(US)**
Inventor: **Garsky, Victor M.**
**752 Palmer Place**
**Blue Bell, PA 19422(US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

PROCEEDINGS OF THE NATIONAL ACADEMY OF SCIENCES USA, vol. 83, August 1986, pages 5708-5712; Z.M. RUGGERI et al.: "Inhibition of platelet function with synthetic peptides designed to be high-affinity antagonists of fibrinogen binding to platelets"

THE JOURNAL OF BIOLOGICAL CHEMISTRY, vol. 263, no. 36, 25th December 1988, pages 19827-19832, The American Society for Biochemistry and Molecular Biology, Inc.; G. ZHONG-RU: "Echistatin: A potent platelet aggregation inhibitor from the venom of the viper Echis carinatus"

Inventor: **Gan, Zhong-Ru**
**Apt. 8-F**
**North Wales, PA 19454(US)**

(74) Representative: **Hesketh, Alan, Dr.**
**European Patent Department**
**Merck & Co., Inc.**
**Terlings Park**
**Eastwick Road**
**Harlow Essex, CM20 2OR (GB)**

## Description

### FIELD OF THE INVENTION

The invention relates to polypeptides for inhibiting platelet aggregation.

### BACKGROUND OF THE INVENTION

This invention relates generally to modulating cell adhesion and to inhibiting the binding of fibrinogen and other proteins to blood platelets, and inhibiting the aggregation of blood platelets. Fibrinogen is a glycoprotein, present in blood plasma, which participates in platelet aggregation and in fibrin formation. Platelets are cell-like anucleated fragments, found in the blood of all mammals, which participate in blood coagulation. Interaction of fibrinogen with a receptor, the platelet membrane glycoprotein complex IIb/IIIa, is known to be essential for normal platelet function.

When a blood vessel is damaged, platelets adhere to the disrupted subendothelial surface. The adherent platelets subsequently release biologically active constituents and aggregate. Aggregation is initiated by the binding of agonists, such as thrombin, epinephrine, or ADP to specific platelet membrane receptors. Stimulation by agonists results in exposure of latent fibrinogen receptors on the platelet surface, and binding of fibrinogen to the glycoprotein IIb/IIIa complex.

Attempts have been made to use natural products and synthetic peptides to study the mechanism of platelet aggregation and adhesion. Platelet aggregation inhibitor proteins have been isolated from a variety of viper venoms. However, studies of the mechanism of inhibition of platelet aggregation by these proteins have been hampered by the lack of structural information.

Recently, a venom peptide called trigramin was characterized in some detail (see Huang et al., J. of Biol. Chem., 1987, 262, pp. 16157-16163 and U.S. Serial No. 121,972, filed November 18, 1987). The peptide was reported to competitively inhibit fibrinogen binding to the glycoprotein IIb/IIIa complex on the platelet membrane. The peptide contains an Arg-Gly-Asp sequence which is believed to be close to the COOH-terminal residue.

Trigramin is disclosed in EP-A-0 317 053, which document constitutes prior art pursuant to Articles 54-(3) and (4) EPC only.

Rouslahti and Pierschbacher, Science, 1987, 238, pp. 491-497, describe adhesive proteins such as fibronectin, vitronectin, osteopontin, collagens, thrombospondin, fibrinogen, and von Willebrand factor present in extracellular matrices and in the blood. The proteins contain the tripeptide arginine-glycine-aspartic acid as their cell recognition site. The tripeptides are recognized by at least one member of a family of structurally related receptors, integrins, which are heterodimeric proteins with two membrane-spanning subunits. The authors state that the conformation of the tripeptide sequence in the individual proteins may be critical to recognition specificity.

Cheresh, Proc. Nat'l. Acad. Sci. USA, 1987, 84, pp. 6471-6475, describes an Arg-Gly-Asp directed adhesion receptor expressed by human endothelial cells that is structurally similar to the IIb/IIIa complex on platelets but antigenically and functionally distinct. The receptor is directly involved in endothelial cell attachment to fibrinogen, von Willebrand factor, and vitronectin.

Pierschbacher and Rouslahti, J. of Biol. Chem.,1987, 262, 36, pp. 17294-17298 describe influence of stereochemistry of the sequence Arg-Gly-Asp-Xaa on binding specificity of peptides containing the tripeptide sequence Arg-Gly-Asp. In Proc. Nat'l. Acad. Sci. USA, 1984, 81, pp. 5985-5988, the same authors describe variants of the cell recognition site of fibronectin that retain attachment-promoting activity. The tetrapeptide Arg-Gly-Asp-Ser is described as the minimal structure recognized by cells in the large, adhesive glycoprotein fibronectin. Peptides having portions -Arg-Gly-Asp-Ser- are described in U.S. Patent Nos. 4,589,881 and 4,614,517. Peptides having portions -Arg-Gly-Asp-R wherein R is selected from Thr or Cys or other amino acid having the same cell-attachment activity as fibronectin, are described in U.S. Patent No. 4,578,079.

Ruggeri et al., Proc. Nat'l. Acad. Sci. USA, 1986, 83, pp. 5708-5712, describes a series of synthetic peptides, designed in lengths to 16 residues, that contain the sequence Arg-Gly-Asp-Val, which inhibit fibrinogen binding to platelets.

Gold et al. Circulation, 77, 3, March 1988, pp 670-677 describes the effects of bolus injections of recombinant single-chain tissue-type plasminogen activator and of $F(ab')_2$ fragments of a murine monoclonal antibody (7E3) against the human platelet GPIIb/IIIa receptor on coronary arterial thrombolysis and reocclusion. Gold et al. found that injection of $F(ab')_2$ fragments of the monoclonal antibody 7E3, directed against the platelet GPIIb/IIIa receptor, accelerates thrombolysis with recombinant single-chain tissue-type

plasminogen activator and prevents reocclusion.

While it is known that the tripeptide sequence Arg-Gly-Asp is present in certain polypeptides which can duplicate or inhibit the cell attachment-promoting effects of fibronectin and vitronectin, there is little understanding of the influence on binding specificity of other amino acids in the polypeptide. Applicants have identified and purified polypeptides which contain the tripeptide sequence Arg-Gly-Asp, which are platelet aggregation inhibitors and which have cysteine residues specifically positioned in relation to the position of the tripeptide. The specific location of the cysteine amino acids appears to significantly influence the ability of these polypeptides to inhibit platelet aggregation.

The advent of reliable methods and equipment for the chemical synthesis of long oligodeoxynucleotides has made gene synthesis a feasible alternative to the isolation and expression of natural genes (Caruthers, Science, 230 (1985) pp. 281-285; Ferretti et al., Proc. Nat'l. Acad. Sci. USA, 83 (1986), pp. 599-603; Wosnick et al., Gene, 60 (1987) pp. 115-127; Nassal, Gene, 66 (1988) pp. 279-294; Bell et al., Gene, 63 - (1988) pp. 155-161). The technique is especially useful for expressing and engineering small natural proteins. However, many eukaryotic proteins expressed in a bacterial host are not stable (Taniguchi et al., Proc. Nat'l. Acad. Sci. USA, 77 (1980) pp 5230-5233; Davis et al., Proc. Nat'l. Acad. Sci. USA, 78 (1981) pp. 5376-5380; Shen, Proc. Nat'l. Acad. Sci. USA, 81 (1984) pp. 4627-4631; and Lennick et al., Gene, 61 (1987) pp. 103-112). A frequent strategy used to circumvent the instability is to fuse the gene of interest to a nucleotide sequence coding for a bacterial protein to produce a fused protein, thereby protecting the eukaryotic protein. Many chemical reagents and proteases have been reported to cleave a variety of amino acid sites on fusion proteins. Cyanogen bromide efficiently and selectively cleaves at methionine (Savige et al., Methods in Enzymology, Hirs and Timashelff eds. Academic Press, New York, 47 (1977) pp. 459-469; Nagai et al., Nature, 309 (1984) pp. 810-812; Szoka et al., DNA, 5 (1986) pp. 11-20; and Haffey et al., DNA, 6 (1987) pp. 565-571).

## SUMMARY OF THE INVENTION

The present invention comprises fibrinogen-receptor antagonist polypeptides characterized in that they inhibit fibrinogen-platelet binding and have the general formula I:

X-Cys-R-R-R-Arg-Gly-Asp-R-R-R-R-R-Cys-Y (I)

wherein X is H or at least one amino acid; Y is OH or at least one amino acid; and each R, either the same or different, is any amino acid.

Polypeptides within the formula I include those of formula Ia:

$H_2N$-(Ch)-Cys-R-R-R-Arg-Gly-Asp-R-R-R-R-R-Cys-(Cx)-H (Ia)

wherein Ch represents at least one amino acid; Cx represents at least one amino acid; and each R-, either the same or different, represents an amino acid.

Polypeptides of the invention have been purified from the venom of various vipers, e.g. Echis carinatus, Agkistrodon piscivorus, Bitis arietans and Eristocophis macmahonii. These polypeptides are useful for inhibiting fibrinogen binding to human platelets and inhibiting fibrinogen-induced aggregation of human platelets.

These polypeptides are purified from viper venom according to the purification procedure described below. They are rich in cysteine and contain the common sequence -Cys-R-R-R-Arg-Gly-Asp-R-R-R-R-R-Cys-, where each R, either the same or different, represents any amino acid.

Although Applicants do not wish to be bound to any particular theory regarding binding mechanism, it is believed that the location of the cysteine amino acids plays an important role in determining the three-dimensional conformation, degree of rigidity and binding specificity of the polypeptides.

The present invention also comprises a gene or degenerate equivalent encoding a platelet aggregation inhibitor of the general formula:

X-Cys-R-R-R-Arg-Gly-Asp-R-R-R-R-R-Cys-Y

wherein X is H or at least one amino acid; Y is OH or at least one amino acid; and each R, either same or different, is any amino acid.

A preferred gene of the present invention encodes modified echistatin. Echistatin is defined as:

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Asp-Met-Asp-Asp-Tyr-Cys-Asn-Gly-Lys-Thr-
Cys-Asp-Cys-Pro-Arg-Asn-Pro-His-Lys-Gly-Pro-Ala-
Thr

The methionine at position 28 of the native protein is replaced by leucine in recombinant echistatin. The synthetic gene is expressed in E. coli (MB-5385, ATCC Accession No. 67873, deposited with American Type Culture Collection, 12301 Parklawn Drive, Rockville, MD 20852 U.S.A.) using an expression vector pJC264, which was constructed by inserting portions of the E. coli cheB and cheY gene complex into the plasmid pUC13 (U.S. Serial No. 145,800, filed November 5, 1988). Microorganisms of the strain have been deposited under the Budapest Treaty and any restrictions on the availability to the public of the deposited microorganism will be irrevocably removed upon the granting of the patent. High level expression of the r-leu 28-echistatin gene was achieved by fusion with the E. coli cheY gene in the expression vector. Recombinant-leu 28-echistatin was liberated from the fusion protein by cyanogen bromide cleavage at the methionine genetically inserted between the cheY protein and echistatin,and purified to homogeneity by reverse phase HPLC, and refolded. The refolded r-leu 28-echistatin is indistinguishable from native echistatin in inhibiting platelet aggregation, suggesting that the methionine at position 28 is not essential for the biological activity of this platelet aggregation inhibitor.

The invention also includes compositions for inhibiting platelet aggregation using proteins of the invention and expression vectors useful for production of recombinant proteins of the invention.

The invention also includes synthetic polypeptides of formula I and methods of synthesis.

## BRIEF DESCRIPTION OF THE FIGURES

### Fig. 1 - Nucleotide and Amino Acid Sequences of Recombinant-leu 28-Echistatin

Individual synthetic oligodeoxynucleotides were marked by solid lines and numbered in brackets. The first amino acid, methionine, at N-terminal protein sequence was designed for cyanogen bromide cleavage. The stop codon at the C-terminus of the protein is indicated by an asterisk EcoRI sites are located at both 5′ and 3′ ends for insertion into the expression vector.

### Fig. 2 - Structure of pJC264 Expression Vector

The construction of the pJC264 expression vector and insertion of the r-leu 28-echistatin gene into the vector are discussed in the Detailed Description of the Invention. A. The structure of pJC264. The sequence derived from pUC13 is indicated by arrows. The E. coli lac promotor-operator in pUC13 precedes the cheB gene and the direction of transcription is indicated by an arrow. The cheB gene (450 bp) and cheY gene (263 bp) are shown as double lines and a shaded box, respectively. The EcoRI site into which the r-leu 28-echistatin gene was inserted is shown. B. The DNA sequence between the cheY PstI site and the EcoRI cloning site, showing the encoded amino acids.

### Fig. 3 - SDS PAGE Analysis of Bacterial Production of Recombinant-leu 28-Echistatin Fusion Proteins

E. coli JM109 containing the pJC264-echistatin expression vector was grown in LB medium with 100 µg/ml ampicillin. When the cell-density reached a $OD_{600}$ reading of 0.2, induction of the expression was started by adding IPTG to the medium to a final concentration of 1mM. Aliquots of the cultures were taken and centrifuged in an Eppendorf centrifuge at different times after induction. The pellets were suspended in SDS PAGE sample buffer containing 50 mM tris-HCl, pH 6.8, 0.2% SDS, 75 mM dithiothreitol, 10% glycerol, and 0.05% bromphenol blue. The samples were heated at 100 °C for 15 min. and then centrifuged in an Eppendorf centrifuge before loading onto an 14% SDS polyacrylamide gradient gel. Lysates equivalent to 100 µl of saturated culture ($OD_{600}$ = 1.8) were analyzed on the gel. The proteins were detected by Coomassie blue staining. Lane F-J: Lysates from cultures induced for 0, 2h, 4h, 6h, and 16h,

respectively; Lane E: Sonicated pellet from about 150 µl culture after 16h induction; Lane D: Lysate from 100 µl of saturated culture from cells transformed with control pJC264 expression vector lacking the r-leu 28-echistatin gene; Lane B: 8 µg of native echistatin; Lane C: 8 µg of purified recombinant-leu 28-echistatin; Lane A: Pharmacia low molecular weight markers (the molecular weights were indicated in the figure); Lane K: Bio-Rad low molecular weight standards, molecular weights from top to bottom correspond to 92.5, 66.2, 45.0, 31.0, 21.0 and 14.4 kDa respectively.

Fig. 4 - HPLC Profiles in the Purification of Recombinant-leu 28-Echistatin and Comparison with Native Echistatin

The purification and HPLC conditions are described in the Detailed Description of the Invention. A: Reduced cyanogen bromide cleavage mixture; B: Native echistatin which was reduced under the same conditions used for the cyanogen bromide cleavage mixture; C: renaturing mixture of purified recombinant-leu 28-echistatin from A; D: Native echistatin. The arrows in panels A and B indicate reduced recombinant-leu 28- and native echistatin, respectively. The position of refolded recombinant-leu 28-echistatin and native echistatin are shown by the arrows in panels C and D, respectively.

Fig. 5 - Inhibition of Platelet Aggregation by Recombinant (r-leu 28-echistatin) and Native (n-echistatin) Echistatin

The assay conditions for platelet aggregation are described in the Detailed Description of the Invention. A: Aggregation was monitored in the absence and presence of 1.5 µM recombinant-leu 28- or native echistatin. Note that the initial decrease in light transmittance, indicative of platelet shape change, was not affected. B: Initial rate of aggregation were measured after addition of ADP in the presence of purified recombinant (leu 28) (squares) echistatin and native (circles) echistatin.

DETAILED DESCRIPTION OF THE INVENTION

Native proteins as used herein refers to full length proteins produced by the corresponding genes. Recombinant proteins refers to the isolation of a gene or cDNA for a desired protein and the use of that purified gene or cDNA to construct a cell which will overproduce the desired protein. Microheterogeneous forms as used herein refers to a single gene product, that is a protein produced from a single gene unit of DNA, which is structurally modified following translation. These structural modifications, however, do not result in any significant alterations of the activity of the protein. The modifications may take place either in vivo or during the isolation and purification process. In vivo modification may result in, but is not limited to, acetylation at the N-terminus, proteolysis, glycosylation or phosphorylation. Proteolysis may include exoproteolysis wherein one or more terminal amino acids are sequentially, enzymatically cleaved to produce microheterogeneous forms which have fewer amino acids than the original gene product. Proteolysis may also include endoproteolytic modification that results from the action of endoproteases which cleave the peptide at specific locations within the amino acid sequence. Similar modifications can occur during the purification process which may result in the production of microheterogeneous forms. The most common modification occuring during purification is proteolysis.

Recombinant DNA technology is defined herein as technology which allows segments of genetic information, DNA, from different cells, usually from different organisms, to be joined end-to-end outside the organisms from which the DNA was obtained and to incorporate this hybrid DNA into a cell that will allow the production of the protein for which the original DNA encodes. Genetic information, DNA or mRNA, is isolated and incorporated into an appropriate cloning vector, and transduced into an appropriate host cell.

Cloning vector as used herein is defined as a DNA sequence which allows the incorporation of specific experimental foreign DNA, with the combined DNA being introduced into a host cell that can exist in a stable manner and express the protein dictated by the experimental DNA. The foreign DNA combined with the vector DNA constitutes a recombinant DNA molecule which is derived from recombinant technology. Cloning vectors may include plasmids, bacteriophage, viruses and cosmids. Expression vectors are defined herein as DNA sequences that are required for the transcription of cloned copies of genes and the translation of their mRNAs in an appropriate host. Such vectors can be used to express either procaryotic or eucaryotic genes in a variety of cells such as bacteria, yeast, insect and mammalian cells. The proteins may also be expressed in a number of virus systems. An appropriately constructed expression vector should contain: an origin of replication for autonomous replication in host cells, selective markers, a limited number of useful restriction enzyme sites, a high copy number, and strong promoters. A promoter is

defined as a DNA sequence that directs RNA polymerase to bind to DNA and to initiate RNA synthesis. A strong promoter is one which causes mRNAs to be initiated at high frequency. Expression vectors may include, but are not limited to, cloning vectors, modified cloning vectors, specifically designed plasmids or viruses.

The proteins of the present invention may exist as, but are not limited to, the complete proteins specified by the defined genes of the native protein or as any fragment or subunit thereof, or as hybrids of the complete protein or its fragments or subunits, so long as they retain platelet aggregation inhibiting activity. The complete proteins, as used herein, refers to the full length polypeptide produced by the appropriate genes. The complete proteins may be obtained by purification of the appropriate viper venom or by expression in an appropriate expression vector of the corresponding recombinant derived gene product. Protein fragments or subunits refers to any portion of the protein which contains fewer amino acids than the complete protein and retains the ability to inhibit platelet aggregation under the same conditions as does the native protein. Hybrid proteins include, but are not limited to, fusion proteins or proteins resulting from the expression of multiple genes within the expression vector. A fusion protein is defined as one in which a limited number of amino acids coded for by the expression vector are expressed and the expression results in their attachment to the specific platelet aggregation inhibiting polypeptide. Proteins resulting from multiple genes may include the specific polypeptide linked to a second polypeptide or peptides by peptide bonds that enhance inhibition of platelet aggregation.

Polypeptides within the scope of the present invention have been isolated from viper venom of Echis carinatus and Agkistrodon piscivorus, Bitis arietans and Eristocophis macmahonii. All of these polypeptides are potent platelet aggregation inhibitors.

The sequence for an inhibitor isolated from Echis carinatus venom is:

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Asp-Met-Asp-Asp-Tyr-Cys-Asn-Gly-Lys-Thr-
Cys-Asp-Cys-Pro-Arg-Asn-Pro-His-Lys-Gly-Pro-Ala-
Thr

This inhibitor is hereinafter referred to as Echistatin.

One sequence for an inhibitor isolated from Bitis arietans venom is:

Ser-Pro-Pro-Val-Cys-Gly-Asn-Glu-Leu-Leu-Glu-Glu-
Gly-Glu-Glu-Cys-Asp-Cys-Gly-Ser-Pro-Ala-Asn-Cys-
Gln-Asp-Arg-Cys-Cys-Asn-Ala-Ala-Thr-Cys-Lys-Leu-
Thr-Pro-Gly-Ser-Gln-Cys-Asn-His-Gly-Glu-Cys-Cys-
Asp-Gln-Cys-Lys-Phe-Lys-Lys-Ala-Arg-Thr-Val-Cys-
Arg-Ile-Ala-Arg-Gly-Asp-Trp-Asn-Asp-Asp-Tyr-Cys-
Thr-Gly-Lys-Ser-Ser-Asp-Cys-Pro-Trp-Asn-His

The inhibitor is hereinafter identified as Bitistatin 1.

Another sequence for an inhibitor isolated from Bitis arietans venom is:

Ser-Pro-Pro-Val-Cys-Gly-Asn-Lys-Ile-Leu-Glu-Gln-
Gly-Glu-Asp-Cys-Asp-Cys-Gly-Ser-Pro-Ala-Asn-Cys-
Gln-Asp-Gln-Cys-Cys-Asn-Ala-Ala-Thr-Cys-Lys-Leu-
Thr-Pro-Gly-Ser-Gln-Cys-Asn-His-Gly-Glu-Cys-Cys-
Asp-Gln-Cys-Lys-Phe-Lys-Lys-Ala-Arg-Thr-Val-Cys-
Arg-Ile-Ala-Arg-Gly-Asp-Trp-Asn-Asp-Asp-Tyr-Cys-
Thr-Gly-Lys-Ser-Ser-Asp-Cys-Pro-Trp-Asn-His.

The inhibitor is hereinafter identified as Bitistatin 2.

Another sequence for an inhibitor isolated from Bitis arietans venom is:

Val-Ser-Pro-Pro-Val-Cys-Gly-Asn-Lys-Ile-Leu-Glu-
Gln-Gly-Glu-Asp-Cys-Asp-Cys-Gly-Ser-Pro-Ala-Asn-
Cys-Gln-Asp-Gln-Cys-Cys-Asn-Ala-Ala-Thr-Cys-Lys-
Leu-Thr-Pro-Gly-Ser-Gln-Cys-Asn-His-Gly-Glu-Cys-
Cys-Asp-Gln-Cys-Lys-Phe-Lys-Lys-Ala-Arg-Thr-Val-
Cys-Arg-Ile-Ala-Arg-Gly-Asp-Trp-Asn-Asp-Asp-Tyr-
Cys-Thr-Gly-Lys-Ser-Ser-Asp-Cys-Pro-Trp-Asn-His.

The inhibitor is hereinafter identified as Bitistatin 3.

The sequence for an inhibitor isolated from Eristocophis macmahonii venom is:

Gln-Glu-Glu-Pro-Cys-Ala-Thr-Gly-Pro-Cys-Cys-Arg-
Arg-Cys-Lys-Phe-Lys-Arg-Ala-Gly-Lys-Val-Cys-Arg-
Val-Ala-Arg-Gly-Asp-Trp-Asn-Asp-Asp-Tyr-Cys-Thr-
Gly-Lys-Ser-Cys-Asp-Cys-Pro-Arg-Asn-Pro-Trp-Asn-
His.

The inhibitor is herinafter identified as Eristostatin.

Another sequence for an inhibitor isolated from Bitis arietans venom is:

Ser-Pro-Pro-Val-Cys-Gly-Asn-Glu-Ile-Leu-Glu-Gln-
Gly-Glu-Asp-Cys-Asp-Cys-Gly-Ser-...

The identity of the sequence following the 20th amino acid has not been determined. Because of the structural similarity between this sequence, hereinafter referred to as Bitistatin 4, and the other polypeptides isolated from Bitis arietans venom, it is believed that Bitistatin 4 falls within the scope of formula I.

The sequences identified above are specific examples of polypeptides falling within the sequence defined in formula I, and should not be interpreted as limiting the scope of the present invention. They have been defined by means of amino acid sequencing. It will be understood that natural allelic variations exist. These variations may be demonstrated by (an) amino acid difference(s) in the overall sequence or by deletions, substitutions, insertions, inversions or additions of (an) amino acid(s) in said sequence.

EXAMPLE 1

METHODS

Materials - The venom of Echis carinatus was purchased from Miami Serpentarium Laboratories, Salt Lake City, UT. Low molecular weight peptide standards, Sephadex G-50, and Mono S FPLC columns were from Pharmacia, Inc. Servalyt Precote isoelectric focusing gels were purchased from Serva Fine Biochemicals, Inc., and isoelectric focusing marker proteins were from BDH Chemicals, Ltd. C18 HPLC columns were the products of Vydac.

Preparation of Platelets

Rabbit whole blood was collected from New Zealand white rabbits. Blood was centrifuged at 200 x g for 10 min. The supernatant, platelet rich plasma, was centrifuged for 15 min at 800 x g in the presence of apyrase (20 µg/ml) and $PGE_1$ (5 ng/ml). The pellet was resuspended in 10 ml of wash buffer (138 mM NaCl, 2.9 mM KCl, 0.31 mM $Na_2HPO_4$, 1 mM $MgCl_2$, 5 mM HEPES, pH 6.5, 5 mM glucose, 0.1% $NaHCO_3$, 1 mM EGTA and 0.35% bovine serum albumin). Apyrase was added to a final concentration of 20 µg/ml. The resuspended pellet was centrifuged at 800 x g for 15 min, then washed a second time in the same buffer. The washed platelets were then suspended in wash buffer, pH 7.4, with EGTA omitted at a concentration of 2-5 x $10^8$ cells/ml.

Platelet Aggregation Assay

Platelet aggregation was measured at 37°C in an aggregometer. The reaction mixture contained washed platelets (2-5 x $10^8$ cells/ml), fibrinogen (100 µg/ml), $Ca^{2+}$ (1 mM), ADP (10 µM), epinephrine (2 µg/ml), and the platelet aggregation inhibitor fraction to be tested. The aggregation was initiated by adding ADP and epinephrine 1 min after the other components had been added. The reaction was allowed to proceed for at least 2 min. The extent of inhibition of aggregation was expressed as the percentage of aggregation observed in the absence of inhibitor.

Purification of Echistatin

Rabbit platelet aggregation as says were used to monitor the activity during purification. Echis carinatus lyophilized venom (1g) was dissolved in 12 ml of 10 mM ammonium bicarbonate, pH 7.7 containing 20 mM DTT. After 15 min at room temperature, the solution was centrifuged at 45,000 x g for 45 min. The pellet was discarded and the supernatant was loaded onto a 2.5 x 125 cm column of Sephadex G-50 equilibrated and eluted at 4° with 10 mM MES, pH 5.3. Fractions containing platelet aggregation inhibitory activity were pooled and concentrated under vacuum. After desalting on a column of Sephadex G-15, 10-13 mg of protein were loaded onto a Mono S FPLC column which had been equilibrated with 10 mM MES, pH 5.3. The bound protein was fractionated at 4° with a linear gradient from 0 to 0.3 M NaCl to 10 mM MES, pH 5.3. The flow rate was 0.6 ml/min. Inhibitory activity eluted as a single peak at about 0.17 M NaCl. The pooled activity was loaded directly onto a C18 reverse phase HPLC column which had been equilibrated with 0.1% (v/v) trifluoroacetic acid in water. The column was developed at room temperature with a polyphasic acetonitrile gradient in 0.1% aqueous TFA, at a flow rate of 0.7 ml/min. Inhibitory activity eluted as a single peak at an acetonitrile concentration of 17%. Volatile material was removed by vacuum centrifugation followed by lyophilization. The resulting protein was homogeneous as judged by SDS polyacrylamide gel electrophoresis, isoelectric focusing, rechromatography on reverse phase HPLC, and N-terminal sequence determination. The yield of purified protein, which we have named Echistatin, was 2.2 mg from 1 g. of starting material.

SDS Polyacrylamide Gel Electrophoresis

We used a modification of the Laemmli system (Nature, 1970, 227, pp. 680-685, hereby incorporated by reference). Stacking and separating gels (1.5 mm) contained 8% and 20% polyacrylamide, respectively. Urea (8 M) was added to the separating gel. The gels were run at 85 volts for 1.5 hr and then at 90 volts for 17 hr. Protein was detected by Coomassie brilliant blue R-250 staining.

Isoelectric Focusing

A Servalyt Precote isoelectric focusing gel was used. Samples were run at a constant power of 1 W in an LKB Ultraphore apparatus. The gels were stained with Serva Blue W.

Reduced and Carboxymethylated Echistatin

Echistatin (0.75 mg protein) was reduced for 1 hr at room temperature with 20 mM DTT in the presence of 0.28 M Tris and 6.7 M guanidine hydrochloride, in a volume of 0.6 ml. Iodoacetic acid (0.06 ml) was then added to give a final concentration of 0.136 M and alkylation was allowed to proceed for 20 min at room temperature. The reduced, carboxymethylated Echistatin was isolated from reagents by C18 reverse phase HPLC.

Chymotryptic Cleavage of Echistatin

Echistatin was digested with chymostrypsin at a ratio of substrate to protease of 40:1 (w/w). The proteolytic reaction was carried out for 4 hr at 37 °C in 0.2 M ammonium bicarbonate, pH 7.8. The reaction mixture was then lyophilized, and chymotryptic peptides were isolated by reverse phase HPLC.

Amino Acid Sequence Determination

Purified Echistatin and selected chymotryptic fragments were subjected to automated protein sequence analysis in an Applied Biosystems 470A Sequencer using the sequencer program and reagents supplied by the manufacturer. Released amino acid derivatives were identified with the aid of an on-line HPLC system.

Protein Determination

Protein was determined by the method of Lowry et al. (J. of Biol. Chem., 1951, 193, pp. 265-275, hereby incorporated by reference) using bovine serum albumin as standard.

RESULTS

Purification

Echistatin was purified to homogeneity from the soluble portion of the lyophilized venom of Echis carinatus in three chromatographic steps. Due to the presence of platelet aggregation stimulatory activity in the crude venom, the inhibitor could not be reliably detected in this fraction. Following gel filtration on Sepahdex G-50, however, a single peak of inhibitory activity was observed. This activity was further purified by cation exchange FPLC on Mono S, and finally by C18 reverse phase HPLC.

One gram of lyophilized venom yielded 2.2 mg of purified Echistatin. This material exhibited a single band on SDS polyacrylamide gel electrophoresis in the presence of 8M urea and on isoelectric focusing. Rechromatography on reverse phase HPLC resulted in a single symmetrical absorbance peak which corresponded exactly with platelet aggregation inhibitory activity. Further confirmation of the homogeneity of the Echistatin preparation was obtained during amino acid sequencing of the native or the reduced and carboxymethylated protein. The only amino acid residue observed during the first sequencer cycle was glutamic acid.

Molecular Weight and Isoelectric Point

The apparent molecular weight of reduced Echistatin during electrophoresis on 20% SDS polyacrylamide gels in the presence of 8 M urea was 5,800 daltons. This is similar to the value calculated from the amino acid sequence (see below). Under nonreducing conditions, the protein migrated somewhat more slowly, corresponding to an apparent molecular weight of 7,000 daltons. Surprisingly, when native Echistatin was treated with hydroxyethyl disulfide, the apparent molecular weight was only about 4,000 daltons. A minor band, probably resulting from incomplete derivatization, was observed in the hydroxyethyl disulfide-treated Echistatin.

Both native and disulfide-treated Echistatin exhibited on pI of 8.3. Upon treatment with dithiothreitol, a minor band with a pI of 7.5 was detected in addition to the predominant band at pI 8.3.

Amino Acid Composition

The amino acid composition of Echistatin, obtained after 20 hr of hydrolysis of the reduced, carboxymethylated protein, is shown in Table I. The composition obtained by this analysis was compared with that predicted from the sequence (see below). Most values obtained by these two methods agreed closely. The content of cysteinyl residues found by amino acid composition (6.7) was lower than that predicted by sequence (8). This may have been due to incomplete carboxymethylation of Echistatin.

TABLE I

Comparison of the amino acid composition of Echistatin between the value determined after hydrolysis and that calculated from sequence:

TABLE I

| Amino Acid | Residues per molecule | |
|---|---|---|
| | Acid hydrolysis | Sequence |
| Cysteine | 6.5 | 8 |
| Aspartic acid 1 | 8.4 | 8 |
| Threonine | 2.8 | 3 |
| Serine | 1.2 | 1 |
| Glutamic acid 2 | 3.3 | 3 |
| Proline | 4.2 | 4 |
| Glycine | 5.3 | 5 |
| Alanine | 1.8 | 2 |
| Methionine | 0.6 | 1 |
| Isoleucine | 1.0 | 1 |
| Leucine | 1.1 | 1 |
| Tyrosine | 1.0 | 1 |
| Phenylalanine | 1.0 | 1 |
| Histidine | 1.2 | 1 |
| Lysine | 5.2 | 5 |
| Arginine | 4.1 | 4 |
| Total | 48.7 | 49 |

[1]The value includes asparagine.
[2]The value includes glutamine.

Effect of Echistatin on platelet aggregation in vitro

Platelet aggregation studies indicate that Echistatin is more potent than trigramin in inhibiting human gel filtered platelet aggregation induced by ADP in the presence of 0.1 M mg/ml fibrinogen ($IC_{50}$ = 30 nM vs. 150 nM).

Echistatin inhibits aggregation of human platelets induced by ADP collagen, platelet activating factor or epinephrine at doses 30-300 nM.

Human Platelet Rich Plasma

Blood was drawn into 0.1 vol. of 3.8% sodium citrate by venipuncture from normal human volunteers. Platelet rich plasma (PRP) was prepared by centrifugation at 400 x g for 12 min. Aggregations were performed by adding ADP collagen, platelet activating factor or epinephrine to PRP and measured in a Sienco aggregometer.

Human Gel Filtered Platelets

Blood was drawn into 0.1 volume of acid-citrate-dextrose (85 mM sodium citrate, 64 mM citric acid, 110 mM dextrose) by venipuncture from normal human volunteers. Platelet rich plasma was prepared by centrifugation at 400 x g for 12 min. $PGE_1$ (5 µg/ml) was added and platelets were collected by centrifugation at 800 x g for 12 min. The platelet pellet was resuspended into human platelet buffer (140 mM NaCl, 7.9 mM KCl, 3.2 mM $Na_2HPO_4$, 6 mM HEPES, 2% bovine serum albumin, 0.1% dextrose, pH 7.2) and filtered over Sepharose 2B that was previously equiliberated in human platelet buffer. Platelets were counted and adjusted to 2 x $10^8$/ml with human platelet buffer.

Amino Acid Sequence

The amino acid sequence of Echistatin was obtained by automated Edman degradation of the reduced, carboxymethylated protein. The protein has 49 amino acids with an N-terminal glutamic acid residue and a C-terminal threonine, and is shown above. Cysteine comprised 8 residues, or greater than 16%, of the total amino acids in Echistatin. The sequence was repeated two times with the same result. When native Echistatin was sequenced, no PTH-amino acid peaks were observed during sequencer cycles where cysteinyl residues were expected, further confirming the assignments at these residues. The disulfide status of these cysteinyl residues is not known.

Further confirmation of the sequence was obtained by analysis of chymotryptic peptides isolated from the reduced, carboxymethylated protein (Table II).

**TABLE II**

**Amino Acid Composition of Chymotryptic Peptides of Echistatin**

**The values in parentheses are the numbers of amino acid residues determined by sequence.**

TABLE II

| | C1 | C2 | C3 | C4 | C3 +C4 |
|---|---|---|---|---|---|
| Cys (CM) | 2.6(4) | 0.7(1) | 1.3(3) | | 1.5(3) |
| Asx | 1.3(1) | | 2.7(3) | | 2.9(3) |
| Thr | | 1.0(1) | 1.0(1) | 1.0(1) | 1.4(2) |
| Ser | 1.1(1) | | | | |
| Glu | 2.0(2) | 1.1(1) | | | |
| Gly | 1.2(1) | 1.4(1) | 1.9(1) | 1.2(1) | 2.2(2) |
| Ala | | | | 1.0(1) | 0.8(1) |
| Ile | | 0.6(1) | | | |
| Leu | | 0.9(1) | | | |
| His | | | 1.2(1) | | 1.3(1) |
| Phe | 0.9(1) | | | | |
| Lys | 1.1(1) | 1.9(2) | 1.7(1) | 1.0(1) | 2.2(2) |
| Arg | 1.2(1) | 0.7(1) | 1.1(1) | | 1.1(1) |
| Pro | 1.3(1) | | 1.4(2) | 1.0(1) | 2.5(3) |

Echistatin contains the sequence Arg-Gly-Asp which is common to certain proteins which bind to the glycoprotein IIb/IIIa complex on the platelet surface, at residues 24-26. This sequence is part of a putative platelet binding site on the fibrinogen molecule.

Echistatin exhibited unusual behavior during SDS polyacrylamide gel electrophoresis in the presence of 8M urea. The native protein migrated with an apparent molecular weight of 7,000 daltons, compared with the value of 5,400 daltons obtained from sequence analysis. Under nonreducing conditions, proteins with intrachain disulfide bonds generally bind less than expected amounts of SDS, while prior reduction increases SDS binding to levels seen with proteins lacking intrachain disulfide bonds (Pitt-Rivers et al., Biochem J., 1968, 109, pp. 825-830). Thus the increased mobility of reduced Echistatin during SDS polyacrylamide gel electrophoresis suggests that this protein contains one or more intrachain disulfide bonds in its native conformation.

Treatment of Echistatin with 80 mM dithiothreitol at room temperature for 20 min completely destroyed its inhibitory activity towards platelet aggregation. Under similar conditions, 80 mM hydroxyethyl disulfide treatment led to the loss of 50% of the inhibitory activity. These results, like those of the SDS gel electrophoresis studies, imply that one or more intrachain disulfide bonds play an important role in maintaining the conformation of Echistatin necessary for its inhibitory activity.

EXAMPLE 2

Methods of protein purification and determination used in Example 1 were employed to isolate and identify a platelet aggregation inhibitor present in Agkistrodon piscivorus venom. The protein, whose amino acid sequence is identified above, displayed characteristics of a platelet aggregation inhibiitor.

EXAMPLE 3

Methods of protein purification and determination used in Example 1 were employed to isolate and identify a platelet aggregation inhibitor present in Bitis arietans venom. The protein, whose amino acid

sequence is identified above, displayed characteristics of a platelet aggregation inhibitor.

EXAMPLE 4

Methods of protein purification and determination used in Example 1 were employed to isolate and identify a second platelet aggregation inhibitor present in Bitis arietans venom. The protein, whose amino acid sequence is identified above, displayed characteristics of a platelet aggregation inhibitor.

EXAMPLE 5

Methods of protein purification and determination used in Example 1 were employed to isolate and identify a third platelet aggregation inhibitor present in Bitis arietans venom. The protein, whose amino acid sequence is identified above, displayed characteristics of a platelet aggregation inhibitor.

EXAMPLE 6

Methods of protein purification and determination used in Example 1 were employed to isolate and identify a platelet aggregation inhibitor present in Eristocophis macmahonii venom. The protein whose amino acid sequence is identified abhove, displayed characteristics of a platelet aggregation inhibitor.

Purification of various polypeptides within formula I to chemical homogeneity according to the present invention has permitted amino acid sequencing of the molecules. Based upon the amino acid sequence shown in formula I, one may advantageously prepare a synthetic gene corresponding to a disclosed amino acid sequence and introduce that gene into an appropriate host by appropriate cloning vectors. Alternatively, it is contemplated that the pure polypeptide may be prepared by obtaining the natural gene from venom producing cells, followed by recombination and cloning. It is therefore understood that the scope of the invention is not merely limited to polypeptides isolated by following the chromatographic procedures disclosed herein, or polypeptides falling within the formula I, but also includes these polypeptides as they may be prepared by genetic engineering techniques.

EXAMPLE 7

DNA and Plasmid Construction, and Protein Expression for Recombinant-leu 28-Echistatin

All enzymes and DNA molecular weight markers were from New England Biolabs. Dideoxy DNA sequencing kits were purchased from United States Biochemical Corporation Inc. Competent E. coli Jm109 was purchased from Stratagene. All the other reagents were analytical or electrophoresis grades.

All oligodeoxynucleotides were synthesized on an Applied Biosystems 380B DNA synthesizer using methylphosphoramidite. After deprotection with concentrated ammonia, oligodeoxynucleotides were desalted on a Pharmacia NAP-10 gel filtration column.

All oligodeoxynucleotides were phosphorylated by ATP and T4 polynucleotide kinase except the two oligodeoxynucleotides overhanging on 5′ ends of both strands. The kinased complementary oligodeoxynucleotides were heated to 95°C and then annealed by cooling slowly to room temperature. Ligation of the 3 duplexes was carried out at 14°C for 15 hours in a reaction mixture containing 0.5 nmol of each duplex, 50 mM Tris-HCl, pH 7.8, 10 mM $MgCl_2$, 20 mM dithiothreitol, 1 mM ATP, 50 µg/ml bovine serum albumin, and 4,000 units of T4 DNA ligase in a total volume of 130 µl. The ligation mixture was fractionated by 10% native polyacrylamide gel electrophoresis. The assembled leu 28-echistatin gene was cut from the polyacrylamide gel and electro-eluted. The eluted leu 28-echistatin gene was kinased, extracted by phenol/chloroform, and precipitated by ethanol before it was inserted into the expression vector.

Construction of pJC264 Expression Vector

A 714 bp HindIII-PstI fragment from pLCI-28 (obtained from B. Bachmann, E. coli Genetic Stock Center) containing the 3′-436 bp of the cheB gene and the 5′-263 bp of cheY gene (Matsumura et al., J. Bact., 160 - (1984) pp. 36-41) was cloned into the pUC13 HindIII and PstI sites. In order to move the unique EcoRI site in the polylinker sequence of pUC13 adjacent to the cheY gene, polylinker sequences between the PstI and EcoRI sites were deleted. The resultant expression vector pJC264 contains a unique EcoRI site which allows genes of interest to fuse with the cheY gene (Fig. 2). For other purposes, the HindIII site connecting pUC13 and the cheB gene was converted to a BamHI site by filling in with the Klenow fragment of E. coli

DNA polymerase I and ligation with a synthetic BamHI linker.

The synthetic recombinant leu 28-echistatin gene was (Fig 1.) inserted into the EcoRI site of pJC264 by ligating one third of synthetic leu 28-echistatin gene prepared above with 0.1 ug EcoRI cut pJC264 under the following conditions: 50 mM Tris-HCl, pH 7.8, 10 mM $MgCl_2$, 20 mM dithiothreitol, 1 mM ATP, 50 ug/ml bovine serum albumin, and 800 units of T4 DNA ligase in a total volume of 20 ul. The ligation was carried out for 15 hours at 14°C.

Transformation of the Recipient Bacteria and Identification of the Positive Clones

One third of the pJC264 expression vector containing the inserted r-leu 28-echistatin gene (7 ul) was used to transform 50 ml of JM109 competent cells under standard conditions. The clones containing the r-leu 28-echistatin gene were selected by restriction mapping. The presence of the coding sequence of echistatin was verified by dideoxy sequence analysis.

Cell Growth and Preparation of Fusion Protein Pellet

Cultures were inoculated with an overnight seed culture (1% V/V) and grown in LB medium containing 100 ug amipicillin/ml. When the cell density reached an $0D_{600}$ reading of 0.2 to 0.3, IPTG was added to give a final concentration of 1 mM. Cells were harvested by centrifugation after another 10 h growth. The cell pellet from a 3000 ml culture was suspended in water to a final volume of 50 ml and sonicated at 4°C for 10 min. using a Fisher Sonic Dismembrator, Model 300, with the large tip at maximum power. The sonicated mixture was centrifuged at 20,000 g for 15 min. The pellet was resuspended in 8 ml water and layered onto 50% sucrose in 15 mM Tris-HCl, pH 7.4, and 0.15 M NaCl. Following centrifugation at 13,000 rpm for 30 min. in an SW28 Beckman rotor at 6°C, the pellet containing the insoluble cheY-Leu 28-echistatin fusion protein was resuspended in 6 ml water.

Cleavage of Fusion Protein

The fusion protein-containing pellet from a 1000 ml culture was cleaved with 60 mM cyanogen bromide in 70% formic acid in a total volume of 13 ml. The reaction proceeded for 15 hours at room temperature. The reagents were removed by extensive dialysis and lyophilization.

Purification and Refolding of Recombinant-leu 28-Echistatin

The cleaved fusion protein was first denatured with 8 M guanidine-HCl at 60°C for 5 min., and then reduced with 0.15 M dithiothreitol at room temperature for 10 min. in the presence of 0.35 M tris-base and 6 M guanidine-HCl. The reduced Leu 28-echistatin was purified by C18 reverse phase HPLC with a 0-65% acetonitrile gradient in the presence of 0.1% trifluoroacetic acid. The HPLC purified r-leu 28-echistatin was oxidatively refolded in 0.1 M ammonium acetate at a protein concentration of 0.2 mg/ml. The refolding reaction proceeded for 72 hours at room temperature with vortex mixing about once every 12 hours.

Platelet Aggregation Assay

Platelet aggregation was measured by the increase in light transmission. Briefly, r-leu 28-echistatin was added to the reaction mixture containing gel-filtered human platelets (2 x $10^8$ cells/ml), fibrinogen (100 $\mu$g/ml), and $Ca^{++}$ (1 mM). Platelet aggregation was initiated by adding ADP (final concentration of 10 $\mu$m) 1 min. after the other components had been added. The rate or extent of aggregation was expressed as the percentage of the maximal light transmission obtained in the absence of echistatin.

Flowchart for the Production of Recombinant leu 28-Echistatin

E. coli (harboring plasmid pJC264-echistatin)

Induce w/IPTG
Cell harvest
Cell lysis
Centrifugation

Fusion protein pellet

Cyanogen bromide

CnBr-cleaved fusion protein pellet

Denature (guanidine-HCl)
Reduce (dithiothreitol)

Fully-reduced, cleaved fusion protein pellet

C18 reverse phase HPLC

Fully reduced r-leu 28-echistatin

Reoxidation/refolding
C18 reverse phase HPLC

Purified, oxidized r-leu 28-echistatin

Design and Construction of Synthetic Gene

Native echistatin is a single chain polypeptide of 49 amino acid residues with a pI of 8.3. A synthetic r-leu 28-echistatin gene was designed and fused with the E. coli cheY gene. The primary structures of the platelet aggregation inhibitors isolated from other sources of the venom of vipers have revealed that methionine 28 is not a conserved amino acid in this family of inhibitors (Huang et al., (1987) J. Biol. Chem., 262, pp. 16157-16163). Therefore, methionine 28 of echistatin was replaced by a leucine and a methionine was inserted between the cheY protein and recombinant-leu 28-echistatin to generate a cyanogen bromide cleavage site (Fig. 1). Two EcoRI sticky ends were designed on the flanking ends in order to allow a junction to the expression vector. A stop codon immediately preceding the 3′-flanking EcoRI site was inserted. The synthetic r-leu 28-echistatin gene was assembled from three double strand fragments which contain KpnI and AvaI restriction sites in the joining sites. Preferred E. coli codons were used in the structural gene except where restriction sites were purposely created. The constructed gene was verified by restriction mapping and DNA sequence analysis.

Construction of Expression Vector

In order to express r-leu 28-echistatin in E. coli, the expression vector pJC264 was used. The vector (Fig. 2) contains the gene encoding the amino-terminal 88 amino acids of the E. coli cheY protein, previously shown to be abundantly and stably expressed in E. coli (Matsumura et al., (1984), J. Bact., 160, pp. 36-41). CheY gene expression in this vector is controlled by E. coli lac promoter-operator from pUC13 (Messing, Methods in Enzymology, Wu et al., eds Academic Press, New York, 101, pp. 20-78 (1983)). Following the cheY gene in pJC264, unique PstI and EcoRI sites facilitate the cloning of new open reading frames and their inducible expression as cheYfusion proteins. The synthetic r-leu 28-echistatin gene was inserted into the EcoRI site of pJC264. The structure of the cheY-echistatin expression vector is shown in Fig. 2.

This vector has several advantages over existing expression systems. CheY fusions are often expressed at high levels (see below and Bailey, et al., (1987) J. Indust. Micro., 2, pp. 47-52). The vector is derived from the high copy number plasmid pUC13 (Messing, Methods in Enzymology, Wu, et al. eds, Academic Press, New York, 101 (1983) pp. 20-78) and efficient replication may contribute to the high expression levels observed. CheY fusion proteins are often found as insoluble inclusion bodies. This insolubility can be exploited in the purification of the fusion protein. The insolubility of the protein may also prevent its degradation by intracellular proteases. Because of the small size of the CheY moiety, the mass ratio of the protein of interest is higher than would be achieved by fusion to the larger, more commonly used β-galactosidase. Finally, the absence of cysteine residues in the CheY protein circumvents potential problems of incorrect disulfide bond formation in the fusion protein.

Expression of Recombinant-leu 28-Echistatin

Expression of recombinant-leu 28-echistatin in E. coli JM109 was induced by IPTG as previously described. The expression product at different times after induction was analyzed by 14% SDS polyacrylamide gel electrophoresis. After 2 hours of induction, a polypeptide of the expected molecular weight of the cheY-echistatin fusion protein (14.5 kDa) was detected in the total cellular lysate (Fig. 3). At 6 hours of induction, this protein accumulated to a maximum level accounting for at least 20% of total cell protein. The expressed protein was insoluble after sonication of the cells, suggesting that inclusion bodies were formed during the expression. The sonicated pellet was subjected to cyanogen bromide cleavage for isolation of recombinant-leu 28-echistatin.

Purification and Renaturation of Recombinant-leu 28-Echistatin

The cyanogen bromide cleaved proteins were reduced by an excess of dithiothreitol in the presence of 6 M guanidine-HCl before purification. Fractionation of the reduced proteins by reverse phase HPLC revealed a peak which had a retention time similar to that of reduced native echistatin (Fig. 4A and B). Amino acid sequence analysis of the protein from this peak showed that it contained recombinant-leu 28-echistatin with a purity of more than 90%. Since the reduced echistatin was in 0.1% trifluoroacetic acid (about pH 2) during HPLC, thiol-disulfide exchange reactions within recombinant-leu 28-echistatin were expected to be very slow. Therefore, the lyophilized echistatin was directly refolded in the presence of 0.1 M ammonium acetate without further denaturation and reduction. It has been proposed that renaturation of an inactive protein is facilitated by adding a thiol/disulfide mixture to the renaturation solution (Tam et al., Nature, 309 (1984) pp. 376-378). We have renatured recombinant-leu 28-echistatin with 0.5 mM dithiothreitol and 1 mM oxidized dithiothreitol in the presence of 6 M guanidine-HCl. However, the yield of refolded echistatin was not increased under such conditions. Correctly folded echistatin was isolated from the unfolded species by reverse phase HPLC. The HPLC profile shown in Fig. 4C indicated that more than 30% of the reduced echistatin was correctly folded under the given conditions. The amount of correctly folded pure echistatin obtained by the purification procedure was estimated about 1.5 mg per liter cell culture. Recombinant-leu 28-echistatin was eluted from C18 column at a slightly higher concentration of acetonitrile than native echistatin (Fig. 4C and 4D). This may be the result of replacing methionine 28 in native echistatin with leucine in recombinant-leu 28-echistatin. The N-terminal amino acid sequence of recombinant-leu 28-echistatin was determined by automated Edman degradation. The sequence up to residue 32 from the N-terminus was found to be identical to the native one except for the leucine to methionine substitution at residue 28.

Biological Activity of Recombinant-leu 28-Echistatin

A comparison of biological activity of recombinant-leu 28- and native echistatin is shown in Fig. 5. The inhibition of platelet aggregation by echistatin was measured in the presence of human fibrinogen and $CaCl_2$ after adding ADP. Like native echistatin, the recombinant protein did not affect the initial decrease in light transmittance following addition of ADP (Fig. 5A), suggesting that the inhibitors did not influence platelet activation. Recombinant-leu 28-echistatin and native echistatin inhibited platelet aggregation, measured as either rate or extent of aggregation, with the same $IC_{50}$ (Fig. 5B). Therefore, recombinant-leu 28-echistatin appears to be identical to native echistatin in its ability to affect platelet aggregation. The results suggest that the presence of methionine at position 28 in native echistatin is not essential for the correct folding and biological activity of this platelet aggregation inhibitor.

The potential exists, in the use of recombinant DNA technology, for the preparation of derivative polypeptides variously modified by resultant single or multiple amino acid substitutions, deletions, additions or replacements, for example, by means of site directed mutagenesis of the underlying DNA. All such allelic variations and modifications resulting in derivative polypeptides are included within the scope of this invention so long as the essential, characteristic platelet aggregation inhibiting activity remains unaffected in kind. The polypeptides are prepared (1) having methionine as its first amino acid (present by virtue of the ATG start signal codon insertion in front of the structural gene) or (2) where the methionine is intra- or extracellularly cleaved, having its normally first amino acid, or (3) together with either its signal polypeptide or a conjugated protein other than the conventional signal polypeptide, the signal polypeptide or conjugate being specifically cleavable in an intra- or extracellular environment (see British Patent Application Publication No. 2,007,676A), or (4) by direct expression in mature form without the necessity of cleaving away any extraneous, superfluous polypeptide. The latter is particularly important where a given host may not, or not efficiently, remove a signal peptide where the expression vehicle is designed to express the protein together with its signal peptide. In any event, the thus produced platelet aggregation inhibitor in its various forms, is recovered and purified to a level fitting it for use in the treatment of various vascular conditions or diseases.

Synthetic Polypeptides of Formula I

Polypeptides of the invention may be prepared using solid phase synthesis, such as that described by Merrifield, J. Am. Chem. Soc., 85, 2149 (1964), although other equivalent chemical syntheses known in the art can also be used, such as the syntheses of Houghten, Proc. Natl. Acal. Sci., 82, 5132 (1985). Solid-phase synthesis is commenced from the C-terminus of the peptide by coupling a protected amino acid to a suitable resin, as generally set forth in U.S. Pat. No. 4,244,946, issued Jan. 21, 1982 to Rivier et al., the disclosure of which is incorporated herein by reference. Examples of synthesis of this general type are set forth in U.S. Pat. Nos. 4,305,872 and 4,316,891. Discussion of the solid-phase synthesis of a 41-residue polypeptide is set forth in Science, 213, 1394-1397 (September 1981) in an article by Vale et al., which refers to a more detailed discussion of the synthesis, which appears in an aritcle by Marke et al. in J. Am. Chem. Soc., 103, 3178 (1981).

In synthesizing the polypeptides, the carboxyl terminal amino acid, having its alpha-amino group suitable protected, is coupled to a chloromethylated polystyrene resin or the like. After removal of the alpha-amino protecting group, as by using trifluoroacetic acid in methylene chloride, the next step in the synthesis is ready to proceed. Other standard cleaving reagents and conditions for the removal of specific amino protecting groups may be used, as described in the open literature.

The remaining alpha-amino- and side-chain-protected amino acids are then coupled stepwise in the desired order to obtain an intermediate compound connected to the resin. As an alternative to adding each amino acid separately in the synthesis, some of them may be coupled to one another prior to the addition to the growing solid-phase chain. The selection of the appropriate coupling reagents is within the skill of the art.

Common to chemical syntheses of peptides is the protection of the labile side-chain groups of the various amino acid moieties with suitable protecting groups at that site until the group is ultimately removed after the chain has been completely assembled. Also common is the protection of the alpha-amino group on an amino acid or a fragment while that entity reacts at the carboxyl group followed by the selective removal of the alpha-amino-protecting group to allow subsequent reaction to take place at that location. Accordingly, it is common that, as a step in the synthesis, an intermediate compound is produced which includes each of the amino acid residues located in the desired sequence in the peptide chain with various of these residues having side-chain protecting groups. These protecting groups are then commonly removed

substantially at the same time so as to produce the desired resultant product following purification.

After the desired amino-acid sequence has been completed, the intermediate peptide is removed from the resin support by treatment with a reagent, such as liquid HF, which not only cleaves the peptide from the resin, but also cleaves all the remaining side-chain protecting groups. The polypeptide can then be purified by gel permeation followed by semipreparative HPLC, as described in Rivier et al., Peptides: Structure and Biological Function (1979) pp. 125-128. A purity of at least 93% or higher (based upon all peptides present) is reasonably obtainable and is preferred for clincial testing and/or use. Purity of 98% is practical; however, for certain in vitro applications, lower purity may be acceptable. Accordingly, the polypeptide is considered useful when it is in substantially pure form which, for purposes of this application, means at least about 50 weight percent, based upon all peptides present.

Examples of various polypeptides of the present invention and methods of synthesis are presented below.

EXAMPLE 8

Boc-O-benzylthreonine-PAM resin, Boc protected amino acids and all other reagents required for synthesis on the Applied Biosystems 430 A (ABI) peptide synthesizer were obtained from the manufacturer. Sidechain protected Asp, Glu and His were supplied by Bachem, Inc. The solvents dimethylformamide (DMF) and methylene chloride ($CH_2Cl_2$) were obtained from Burdick and Jackson. Dithiothreitol (DTT) was purchased from Bethesda Res. Labs. Dithioerythritol (DTT) was obtained from Chemical Dynamics. p-cresol and p-thiocresol were obtained from Aldrich Chemical Co.

Echistatin Synthesis

Starting with 0.50 mM (0.69 g) of Boc-Thr(Bzl)O-Pam-resin (substitution at 0.72 mM of Thr/g of resin) the synthesis was carried out in a stepwise manner using the ABI automated peptide synthesizer. Kent and Clark-Lewis (1985) Synthetic Peptides in Biology and Medicine, Proc. Labsystems Res. Symp., Eds. Alitalo et al., Elsevier, Netherlands pp. 29-57. The amino acids were introduced using the manufacturer's prepacked cartridges (2 mM each). Sidechain protection was Arg (Tos), Asp (OcHx), Cys (Meb), Glu (OcHx), His (Bom) Lys[Z(Cl)], Ser (Bzl), Thr (Bzl), Tyr[Z(Br)]. [Tos, Tosyl; cHx, cyclo-hexyl; Meb, 4-methylbenzyl; Z(Cl), 2-chlorobenzyloxycarbonyl; Bom, benzyloxymethyl; Bzl, benzyl; Z(Br), 2 bromobenzyloxycarbonyl]. Double coupling with symmetric anhydrides (performed in $CH_2Cl_2$ followed by solvent exchange with DMF) were used for all Boc-protected amino acids except for Arg (Tos), Asn and His (Bom), where hydroxybenzotriazole esters in DMF were used in a double coupling protocol. In order to protect against undesired acid-catalyzed oxidations of Cys and Met (Draper et al. (1973) J. Med. Chem. Vol. 16, pp. 1326-1329) during trifluoroacetic acid (TFA) deblocking, 0.1% (wt/v) DTE was added as a scavenger. Following the coupling of N-terminal Glu the Boc group was removed using TFA and the peptide-resin was dried. The final weight of N-terminal deblocked peptide-resin was 4.15 g.

HF Cleavage and Oxidation

The assembled peptide-resin (2.0 g) was suspended in a mixture of 3 ml of 1:1 (v:v) p-thiocresol/p-cresol in an HF apparatus (Peninusula Labs. Inc., Type 1B). The system was evacuated with a mechanical vacuum pump and HF condensed (30 mL) using liquid nitrogen cooling. After stirring at 0-5°C for 1 1/2 hr the reaction mixture was evaporated in vacuo using a liquid nitrogen trap (20-30 min). The residue was triturated with ether, filtered and washed 3 times with additional ether. The filtered residue was immediately transferred to 4 liters of a stirred solution of dilute acetic acid (0.4%/$H_2O$). After stirring for several minutes the pH of the mixture was adjusted to 8.0 with ammonium hydroxide. Following filtration to remove resin, the crude oxidation product was maintained without stirring at 5°C (18 hr) and subsequently at ambient temperature (19-20°C) for 3 days. Analytical HPLC was used to monitor the progress of the oxidation. A qualitative Ellman test (Habeeb, Methods in Enzymology (1972), Eds. Hirs and Timasheff, Academic Press New York pp. 457-464) was used to monitor the disappearance of free sulfhydryl groups before proceeding with purification. This test was performed on a 1 ml sample which was lyophilized in order to remove residual p-thiocresol.

Purification of Crude Oxidized Echistatin

The crude oxidized solution (4 l) was acidified by addition of acetic acid (10 ml) and pumped directly onto a $C_{18}$-silica (5 x 30 cm, 15 m, 300 A) cartridge (Waters Associates). The product was purified using preparative HPLC (Separations Technology, Inc.). A step gradient (100 mL increments) which was generated from 1 liter each of successively increasing concentrations of mobile phase. A flow rate of 70 mL/min was used to elute the product. Homogenous (>95%) fractions as determined by RP-HPLC (Vydac $C_{18}$, 218TP5415) were pooled and lyophilized to give 72 mg of product. The semi-pure product was contaminated with a less polar component as a shoulder to the product peak. The product was further purified by repassage on HPLC in the same manner as described above to yield echistatin (54 mg). Based on 0.25 mM of starting resin this weight represents a 4 percent overall yield. Homogeneity was demonstrated by analytical HPLC. Coinjection of synthetic product with native material gave a single peak. Product was further characterized by amino acid analysis after hydrolysis with 6N HCl (Table 3) and by automated Edman degradation (ABI 470A Protein Sequencer).

TABLE 3

| AMINO ACID ANALYSIS[a] OF SYNTHETIC ECHISTATIN (THEORETICAL NUMBER IN PARENTHESIS) | | | |
|---|---|---|---|
| Lys | 5.00 (5) | Gly | 5.03 (5) |
| His | 1.00 (1) | Ala | 2.10 (2) |
| Arg | 3.87 (4) | Cys[d] | 7.67 (8) |
| Asp | 8.13 (8) | Met[c] | 0.84 (1) |
| Thr[b] | 2.99 (3) | Leu | 0.98 (1) |
| Ser[b] | 1.04 (1) | Phe | 0.95 (1) |
| Glu | 3.07 (3) | Tyr | 1.00 (1) |
| Pro | 4.08 (4) | Ile + allo-Ile | 0.99 (1) |

[a]After hydrolysis with 6N HCl at 100°C for 70 hrs.
[b]Corrected for decomposition during hydrolysis.
[c]Uncorrected.
[d]Determined as cysteic acid following performic acid oxidation

A maximum of 1.9 percent preview was observed. The high yield of PTH amino acids from the first step also demonstrated that cyclization of the C-terminal Glu to pyro-Glu had not occurred.

Reduction and Refolding of Synthetic Echistatin

Synthetic echistatin (0.50 mg) was dissolved in 1 ml of 0.07 M pH 8.0 ammonium acetate (10 mM DTT) and the course of reduction followed by analytical HPLC. After 1 hr the starting material was converted quantitatively to a single reduced product. Dialysis (24 hr) of the reduced product using a 12 mm diameter, 1000 MW cutoff, cellulose tubing (Spectrum Medical Ind.) against (4 l) of a 0.07 M ammonium acetate buffer (pH 8.0) produced only echistatin. In order to demonstrate that the echistatin was in its fully reduced form prior to reoxidation, the DTT reduction of synthetic echistatin was repeated as described but in the presence of 6M guanidine hydrochloride. Analytical HPLC confirmed that the reduced products had identical times. Isolation of reduced echistatin, by semi-preparative HPLC, followed by quantitative Ellman analysis (Habeeb, ibid.) showed the product to be in the octahydro form.

Platelet aggregation assay

Platelet aggregation was measured at 37°C in a chronolog aggregometer. The reaction mixture contained washed platelets ($2 \times 10^8$/ml), fibrinogen (100 mg/mL), $Ca^{2+}$ (1 mM), and the platelet aggregation inhibitor fraction to be tested The aggregation was initiated by adding 10 mM ADP 1 min after the other components had been added. The reaction was allowed to proceed for at least 2 min. The extent of inhibition of aggregation was expressed as the percentage of the rate of aggregation observed in the absence of inhibitor. Synthetic echistatin possesses chemical and biological properties indistinguishable from those of natural echistatin (Table 4).

TABLE 4

| EFFECT OF NATIVE ECHISTATIN AND SYNTHETIC ECHISTATIN ON INHIBITION OF HUMAN PLATELET AGGREGATION | |
|---|---|
| PEPTIDE | $IC_{50}$(μM)(95% CONFIDENCE LIMITS) |
| Native Echistatin | 0.032 (0.026-0.039) |
| Synthetic Echistatin | 0.033 (0.026-0.041) |

The concentration of peptide necessary to inhibit the rate of human platelet aggregation by 50% ($IC_{50}$) was determined using platelets from 2-4 donors. Determinations were performed in duplicate for each donor.

EXAMPLES 9-20

Following the general synthesis procedures for preparing echistatin described in Example 8, many polypeptides having sequences similar to echistatin and falling within the general formula I were prepared.

TABLE 5

| ECHISTATIN ANALOGS PREPARED BY SYNTHETIC MEANS, AND THEIR EFFECT ON HUMAN PLATELET AGGREGATION | | |
|---|---|---|
| Example No. | Sequence | $IC_{50}$(μM)(95% CONFIDENCE LIMITS) |
| 9 | phe 27-echistatin | .013 (.010-.016) |
| 10 | phe 27, leu 28-echistatin | .016 (.011-.022) |
| 11 | trp 27-echistatin | .017 (.014-.019) |
| 12 | pro 23, phe 27, leu 28-echistatin | .026 (.021-.033) |
| 13 | leu 28-echistatin | .034 (.026-.043) |
| 14 | phe 28-echistatin | .038 (.026-.058) |
| 15 | asn 22, phe 27, leu 28-echistatin | .038 (.031-.047) |
| 16 | (1-41)-echistatin amide | .066 (.048-.084) |
| 17 | (1-43)-echistatin amide | .081 (.066-.099) |
| 18 | met SO 28-echistatin | .088 (.033-.154) |
| 19 | (1-39)-echistatin amide | .327 (.214-.414) |
| 20 | (1-39)-echistatin | .800 (.580-1.1) |

The abbreviations above define the various synthesized polypeptides. In examples 9-15 and 18, the sequence position number(s) which is (are) substituted and the residue(s) replacing the naturally occuring residue(s) is (are) indicated (e.g. in Example 9, Asp, the residue at position 27 which occurs in natural echistatin, is replaced with Phe). Residues 1-26 and 28-49 are identical to natural echistatin. Examples 16, 17, 19 and 20 are sequences having residues toward the carboxyl terminal end omitted (e.g. in Example 20, residues 40-49 have been omitted). met SO in Example 18 represents methionine sulfoxide.

Therapeutic Utility

Polypeptides of the invention may be administered in any situation where inhibition of human or mammalian platelet aggregation or adhesion is desired.

Polypeptides of the invention are eliminated from circulation rapidly and are particularly useful in inhibiting platelet aggregation in situations where a strong antithrombotic of short duration of effectiveness is needed. Thus, they may find utility in surgery on peripheral arteries (arterial grafts, carotid endarterectomy) and in cardiovascular surgery where manipulation of arteries and organs, and/or the interaction of platelets with artificial surfaces, leads to platelet aggregation and consumption. The aggregated platelets may form thrombi and thromboemboli. Polypeptides of the invention may be administered to these surgical patients to prevent the formation of thrombi and thromboemboli.

Extracorporeal circulation is routinely used for cardiovascular surgery in order to oxygenate blood. Platelets adhere to surfaces of the extracorporeal circuit. Adhesion is dependent on the interaction between GPIIb/IIIa on the platelet membranes and fibrinogen adsorbed to the surface of the circuit. (Gluszko et al., Amer. J. Physiol., 1987, 252:H, pp 615-621). Platelets released from artificial surfaces show impaired hemostatic function. Polypeptides of the invention may be administered to prevent adhesion.

Other applications of these polypeptides include prevention of platelet thrombosis, thromboembolism and reocclusion during and after thrombolytic therapy and prevention of platelet thrombosis, thromboembolism and reocclusion after angioplasty of coronary and other arteries and after coronary artery bypass procedures. In many clinical centers patients subjected to these procedures are already receiving antiplatelet drugs which are weaker inhibitors of platelet aggregation as compared to polypeptides of the present invention. Polypeptides of the invention may also be used to prevent myocardial infarction.

These polypeptides may be administered by any convenient means which will result in its delivery into the blood stream in substantial amount. They may be combined with thrombolytic agents such as plasminogen activators or streptokinase in order to inhibit platelet aggregation. They may also be combined with anticoagulants such as heparin, aspirin or warfarin. Intravenous administration is presently contemplated as the preferred administration route. They are soluble in water, and may therefore be effectively administered in solution.

In one exemplary application, a suitable amount of Echistatin is intravenously administered to a heart attack victim undergoing angioplasty. Administration occurs during or several minutes prior to angioplasty, and is in an amount sufficient to inhibit platelet aggregation, e.g. an amount which achieves a steady state plasma concentration of between about 0.05 - 2 µM. Should the patient need to undergo bypass surgery, Echistatin administration may be stopped immediately and will not cause complications during surgery that would be caused by other materials such as aspirin or monoclonal antibodies (e.g. 7E3, see Gold et al.) the effects of which last hours after cessation of administration.

The present invention also includes a composition comprising recombinant single-chain tissue-type plasminogen activator or streptokinase and a polypeptide having a sequence

$H_2N$-(Ch)-Cys-
R-R-R-Arg-Gly-Asp-R-R-R-R-R-Cys-(Cx)-H,

where each R, either the same or different, represents any amino acid. The invention also includes a method for promoting thrombolysis and preventing reocclusion in a patient which comprises administering to the patient an amount of recombinant single-chain tissue-type plasminogen activator and an amount of a peptide having a sequence

$H_2N$-(Ch)-Cys-R-R-R-Arg-
Gly-Asp-R-R-R-R-R-Cys-(Cx)-H,

where each R, either the same or different, represents any amino acid.

The present invention may be embodied in other specific forms without departing from the spirit or essential attributes thereof. Thus, the specific examples described above should not be interpreted as limiting the scope of the present invention.

**Claims**

**Claims for the following Contracting States : AT, BE, CH, LI, DE, FR, GB, IT, LU, NL, SE**

1. A polypeptide having the following amino acid sequence:

X-Cys-R-R-R-Arg-Gly-Asp-R-R-R-R-R-Cys-Y

wherein X is H or at least one amino acid; Y is OH or at least one amino acid; and each R, either the same or different, is any amino acid; with the exception of Trigramin.

2. A polypeptide according to Claim 1, having the following amino acid sequence:

$H_2N$-(Ch)-Cys-R-R-R-Arg-Gly-Asp-R-R-R-R-R-Cys-(Cx)-H

wherein Ch represents at least one amino acid; Cx represents at least one amino acid; and each R, either the same or different, represents an amino acid.

3. A substantially pure polypeptide of Claim 1 called "Echistatin" having the following amino acid sequence:

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Asp-Met-Asp-Asp-Tyr-Cys-Asn-Gly-Lys-Thr-
Cys-Asp-Cys-Pro-Arg-Asn-Pro-His-Lys-Gly-Pro-Ala-
Thr

4. A substantially pure polypeptide of Claim 1 called "Bitistatin 1" having the following sequence:

Ser-Pro-Pro-Val-Cys-Gly-Asn-Glu-Leu-Leu-Glu-Glu-
Gly-Glu-Glu-Cys-Asp-Cys-Gly-Ser-Pro-Ala-Asn-Cys-
Gln-Asp-Arg-Cys-Cys-Asn-Ala-Ala-Thr-Cys-Lys-Leu-
Thr-Pro-Gly-Ser-Gln-Cys-Asn-His-Gly-Glu-Cys-Cys-
Asp-Gln-Cys-Lys-Phe-Lys-Lys-Ala-Arg-Thr-Val-Cys-
Arg-Ile-Ala-Arg-Gly-Asp-Trp-Asn-Asp-Asp-Tyr-Cys-
Thr-Gly-Lys-Ser-Ser-Asp-Cys-Pro-Trp-Asn-His

5. A substantially pure polypeptide of Claim 1 called "Bitistatin 2" having the following sequence:

Ser-Pro-Pro-Val-Cys-Gly-Asn-Lys-Ile-Leu-Glu-Gln-
Gly-Glu-Asp-Cys-Asp-Cys-Gly-Ser-Pro-Ala-Asn-Cys-
Gln-Asp-Gln-Cys-Cys-Asn-Ala-Ala-Thr-Cys-Lys-Leu-
Thr-Pro-Gly-Ser-Gln-Cys-Asn-His-Gly-Glu-Cys-Cys-
Asp-Gln-Asp-Lys-Phe-Lys-Lys-Ala-Arg-Thr-Val-Cys-
Arg-Ile-Ala-Arg-Gly-Asp-Trp-Asn-Asp-Asp-Tyr-Cys-
Thr-Gly-Lys-Ser-Ser-Asp-Cys-Pro-Trp-Asn-His

6. A substantially pure polypeptide of Claim 1 called "Bitistatin 3" having the following sequence:

Val-Ser-Pro-Pro-Val-Cys-Gly-Asn-Lys-Ile-Leu-Glu-
Gln-Gly-Glu-Asp-Cys-Asp-Cys-Gly-Ser-Pro-Ala-Asn-
Cys-Gln-Asp-Gln-Cys-Cys-Asn-Ala-Ala-Thr-Cys-Lys-
Leu-Thr-Pro-Gly-Ser-Gln-Cys-Asn-His-Gly-Glu-Cys-
Cys-Asp-Gln-Cys-Lys-Phe-Lys-Lys-Ala-Arg-Thr-Val-
Cys-Arg-Ile-Ala-Arg-Gly-Asp-Trp-Asn-Asp-Asp-Tyr-
Cys-Thr-Gly-Lys-Ser-Ser-Asp-Cys-Pro-Trp-Asn-His

7. A substantially pure polypeptide of Claim 1 called "Eristostatin" having the following amino acid sequence:

Gln-Glu-Glu-Pro-Cys-Ala-Thr-Gly-Pro-Cys-Cys-Arg-
Arg-Cys-Lys-Phe-Lys-Arg-Ala-Gly-Lys-Val-Cys-Arg-
Val-Ala-Arg-Gly-Asp-Trp-Asn-Asp-Asp-Tyr-Cys-Thr-
Gly-Lys-Ser-Cys-Asp-Cys-Pro-Arg-Asn-Pro-Trp-Asn-
His

8. A polypeptide according to Claim 1, having the following amino acid sequence:

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Phe-Met-Asp-Asp-Tyr-Cys-Asn-Gly-Lys-Thr-
Cys-Asp-Cys-Pro-Arg-Asn-Pro-His-Lys-Gly-Pro-Ala-
Thr

9. A polypeptide according to Claim 1, having the following amino acid sequence:

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Phe-Leu-Asp-Asp-Tyr-Cys-Asn-Gly-Lys-Thr-
Cys-Asp-Cys-Pro-Arg-Asn-Pro-His-Lys-Gly-Pro-Ala-
Thr

**10.** A polypeptide according to Claim 1, having the following amino acid sequence:

Glu-Cys-Glu-Ser-Gly-Pro-Cys-Cys-Arg-Asn-Cys-Lys-Phe-Leu-Lys-Glu-Gly-Thr-Ile-Cys-Lys-Arg-Ala-Arg-Gly-Asp-Trp-Met-Asp-Asp-Tyr-Cys-Asn-Gly-Lys-Thr-Cys-Asp-Cys-Pro-Arg-Asn-Pro-His-Lys-Gly-Pro-Ala-Thr

**11.** A polypeptide according to Claim 1, having the following amino acid sequence:

Glu-Cys-Glu-Ser-Gly-Pro-Cys-Cys-Arg-Asn-Cys-Lys-Phe-Leu-Lys-Glu-Gly-Thr-Ile-Cys-Lys-Arg-Pro-Arg-Gly-Asp-Phe-Leu-Asp-Asp-Tyr-Cys-Asn-Gly-Lys-Thr-Cys-Asp-Cys-Pro-Arg-Asn-Pro-His-Lys-Gly-Pro-Ala-Thr

**12.** A polypeptide according to Claim 1, having the following amino acid sequence:

Glu-Cys-Glu-Ser-Gly-Pro-Cys-Cys-Arg-Asn-Cys-Lys-Phe-Leu-Lys-Glu-Gly-Thr-Ile-Cys-Lys-Arg-Ala-Arg-Gly-Asp-Asp-Leu-Asp-Asp-Tyr-Cys-Asn-Gly-Lys-Thr-Cys-Asp-Cys-Pro-Arg-Asn-Pro-His-Lys-Gly-Pro-Ala-Thr

**13.** A polypeptide according to Claim 1, having the following amino acid sequence:

Glu-Cys-Glu-Ser-Gly-Pro-Cys-Cys-Arg-Asn-Cys-Lys-Phe-Leu-Lys-Glu-Gly-Thr-Ile-Cys-Lys-Arg-Ala-Arg-Gly-Asp-Asp-Phe-Asp-Asp-Tyr-Cys-Asn-Gly-Lys-Thr-Cys-Asp-Cys-Pro-Arg-Asn-Pro-His-Lys-Gly-Pro-Ala-Thr

**14.** A polypeptide according to Claim 1, having the following amino acid sequence:

Glu-Cys-Glu-Ser-Gly-Pro-Cys-Cys-Arg-Asn-Cys-Lys-Phe-Leu-Lys-Glu-Gly-Thr-Ile-Cys-Lys-Asn-Ala-Arg-Gly-Asp-Phe-Leu-Asp-Asp-Tyr-Cys-Asn-Gly-Lys-Thr-Cys-Asp-Cys-Pro-Arg-Asn-Pro-His-Lys-Gly-Pro-Ala-Thr

**15.** A method of inhibiting fibrinogen binding to mammalian platelets or fibrinogen-induced aggregation of mammalian platelets comprising incubating mammalian platelets with a polypeptide according to Claim 1.

**16.** The use of a polypeptide according to Claim 1 for the manufacture of a medicament useful for inhibiting aggregation of platelets in a mammal.

**17.** A method for purifying a polypeptide of the following sequence:

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Asp-Met-Asp-Asp-Tyr-Cys-Asn-Gly-Lys-Thr-
Cys-Asp-Cys-Pro-Arg-Asn-Pro-His-Lys-Gly-Pro-Ala-
Thr

comprising
(a) dissolving lyophilized Echis carinatus venom in slightly basic solution;
(b) centrifuging the solution to obtain supernatant;
(c) loading supernatant onto a column; and
(d) determining fractions containing platelet aggregation inhibitory activity and concentrating under vacuum.

**18.** A DNA molecule comprising a recombinant DNA encoding a polypeptide having the following amino acid sequence:

$H_2$-(Ch)-Cys-R-R-R-Arg-Gly-Asp-R-R-R-R-R-Cys-(Cx)-H

wherein Ch represents at least one amino acid; Cx represents at least one amino acid; and each R, either the same or different, represents an amino acid, wherein said polypeptide inhibits platelet aggregation.

**19.** A DNA Molecule according to Claim 19, wherein the amino acid sequence is as follows:

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Phe-Leu-Asp-Asp-Tyr-Cys-Asn-Gly-Lys-Thr-
Cys-Asp-Cys-Pro-Arg-Asn-Pro-His-Lys-Gly-Pro-Ala-
Thr

**20.** A replicable microbial expression vector comprising a promoter-operator sequence capable of expressing heterologous proteins in a microorganism followed by DNA encoding a platelet aggregation inhibitor having an amino acid sequence

$H_2N$-(Ch)-Cys-R-R-R-Arg-Gly-Asp-R-R-R-R-R-Cys-(Cx)-H

wherein Ch represents at least one amino acid; Cx represents at least one amino acid; and each R, either the same or different, represents an amino acid, wherein transcription of said DNA in a transformant microorganism is under control of said promoter-operator.

**21.** An expression vector of Claim 21, wherein the amino acid sequence has the following amino acid sequence:

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Asp-Leu-Asp-Asp-Tyr-Cys-Asn-Gly-Lys-Thr-
Cys-Asp-Cys-Pro-Arg-Asn-Pro-His-Lys-Gly-Pro-Ala-
Thr

**22.** A viable cell culture comprising cells transformed with the expression vector of Claim 22.

**23.** A process for preparing a cysteine-rich polypeptide containing the following sequence:

$H_2$N-(Ch)-Cys-R-R-R-Arg-Gly-Asp-R-R-R-R-R-Cys-(Cx)-H

wherein Ch represents at least one amino acid; Cx represents at least one amino acid; and each R, either the same or different, represents an amino acid, wherein:

(a) the C-terminal first amino acid of the sequence is attached to a solid cross-linked polystyrene resin to form an amino acyl polymer, the C-terminal first amino acid blocking group is removed from the amino acyl polymer, the next sequential second amino acid is coupled to the C-terminal amino acid residue, subsequent amino acids are coupled in step-wise fashion to form the polypeptide sequence, and the N-terminal Boc group protecting the N-terminal amino acid is removed;
(b) the polypeptide sequence is treated with HF and one or more thio-containing scavengers;
(c) the treated sequence is washed and transferred from the column to a large volume of dilute acetic acid to minimize undesirable crosslinking; and
(d) the solution resulting from step (c) is adjusted to a pH of about 8.0 stirred.

**Claims for the following Contracting States : ES, GR**

**1.** A process for preparing a cysteine-rich polypeptide having the following aminoacid sequence

X-Cys-R-R-R-Arg-Gly-Asp-R-R-R-R-R-Cys-Y

wherein
X is H or at least one amino acid;
Y is H or at least one amino acid;
and
each R, either the same or different, is any amino acid;
with the exception of Trigramin;
which comprises:

a) Purifying the polypeptide from the venom of vanous viper such as Echis carinatus, Agkistrodon piscivorus, Bitis arietans and Eristocophis macmohonii; or alternatively
b) Expressing the gene that encodes for the desired polypeptide, into an appropiate host by recombinant DNA technology; or alternatively
c) Chemically synthetizing the desired polypeptide by synthesis of Merrifield or by synthesis of Houghten.

**2.** A process according to claim 1, wherein the polypeptide comprises the following sequence

$H_2$N-(Ch)-Cys-R-R-R-Arg-Gly-Asp-R-R-R-R-R-Cys-(Cx)-H

wherein
Ch represents at least one amino acid;

Cx represents al least one amino acid;
and each R, either the same or different, represents an amino acid;
said process is characterized in that:
a) The C-terminal first amino acid of the sequence is attached to a solid cross-linked polystyrene resin to form an amino acyl polymer, the C-terminal first amino acid blocking group is removed from the amino acyl polymer the next sequential second amino is coupled to the C-terminal amino acid residue, subsequent amino acids are coupled in step-wise fashion to form the polypeptide sequence, and the N-terminal Boc group protecting the N-terminal amino acid is removed;
b) The polypeptide sequence is treated with HF and one or more thio-containing scavengers;
c) The treated sequence is washed and transferred from the column to a large-volume of dilute acetic acid to minimize undesirable crosslinking; and
d) The solution resulting from step (c) is adjusted to a pH of about 8.0 stirred.

3. A process according to claim 1, wherein the polypeptide obtained is called "Echistatin" and has the following amino acid sequence:

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Asp-Met-Asp-Asp-Tyr-Cys-Asn-Gly-Lys-Thr-
Cys-Asp-Cys-Pro-Arg-Asn-Pro-His-Lys-Gly-Pro-Ala-
Thr

4. A process according to claim 1, wherein the polypeptide obtained is called "Bitistatin 1" and has the following amino acid sequence:

Ser-Pro-Pro-Val-Cys-Gly-Asn-Glu-Leu-Leu-Glu-Glu-
Gly-Glu-Glu-Cys-Asp-Cys-Gly-Ser-Pro-Ala-Asn-Cys-
Gln-Asp-Arg-Cys-Cys-Asn-Ala-Ala-Thr-Cys-Lys-Leu-
Thr-Pro-Gly-Ser-Gln-Cys-Asn-His-Gly-Glu-Cys-Cys-
Asp-Gln-Cys-Lys-Phe-Lys-Lys-Ala-Arg-Thr-Val-Cys-
Arg-Ile-Ala-Arg-Gly-Asp-Trp-Asn-Asp-Asp-Tyr-Cys-
Thr-Gly-Lys-Ser-Ser-Asp-Cys-Pro-Trp-Asn-His

5. A process according to claim 1, wherein the polypeptide obtained is called "Bitistatin 2" and has the following amino acid sequence:

Ser-Pro-Pro-Val-Cys-Gly-Asn-Lys-Ile-Leu-Glu-Gln-
Gly-Glu-Asp-Cys-Asp-Cys-Gly-Ser-Pro-Ala-Asn-Cys-
Gln-Asp-Gln-Cys-Cys-Asn-Ala-Ala-Thr-Cys-Lys-Leu-
Thr-Pro-Gly-Ser-Gln-Cys-Asn-His-Gly-Glu-Cys-Cys-
Asp-Gln-Asp-Lys-Phe-Lys-Lys-Ala-Arg-Thr-Val-Cys-
Arg-Ile-Ala-Arg-Gly-Asp-Trp-Asn-Asp-Asp-Tyr-Cys-
Thr-Gly-Lys-Ser-Ser-Asp-Cys-Pro-Trp-Asn-His

6. A process according to claim 1, wherein the polypeptide obtained is called "Bitistatin 3" and has the following amino acid sequence:

Val-Ser-Pro-Pro-Val-Cys-Gly-Asn-Lys-Ile-Leu-Glu-
Gln-Gly-Glu-Asp-Cys-Asp-Cys-Gly-Ser-Pro-Ala-Asn-
Cys-Gln-Asp-Gln-Cys-Cys-Asn-Ala-Ala-Thr-Cys-Lys-
Leu-Thr-Pro-Gly-Ser-Gln-Cys-Asn-His-Gly-Glu-Cys-

Cys-Asp-Gln-Cys-Lys-Phe-Lys-Lys-Ala-Arg-Thr-Val-
Cys-Arg-Ile-Ala-Arg-Gly-Asp-Trp-Asn-Asp-Asp-Tyr-
Cys-Thr-Gly-Lys-Ser-Ser-Asp-Cys-Pro-Trp-Asn-His

7. A process according to claim 1, wherein the polypeptide obtained is called "Eristostatin" and has the following amino acid sequence:

Gln-Glu-Glu-Pro-Cys-Ala-Thr-Gly-Pro-Cys-Cys-Arg-
Arg-Cys-Lys-Phe-Lys-Arg-Ala-Gly-Lys-Val-Cys-Arg-
Val-Ala-Arg-Gly-Asp-Trp-Asn-Asp-Asp-Tyr-Cys-Thr-
Gly-Lys-Ser-Cys-Asp-Cys-Pro-Arg-Asn-Pro-Trp-Asn-
His

8. A process according to ciaim 1, wherein the polypeptide obtained has the following amino acid sequence:

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Phe-Met-Asp-Asp-Tyr-Cys-Asn-Gly-Lys-Thr-
Cys-Asp-Cys-Pro-Arg-Asn-Pro-His-Lys-Gly-Pro-Ala-
Thr

9. A process according to claim 1, wherein the polypeptide obtained has the following amino acid sequence:

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Phe-Leu-Asp-Asp-Tyr-Cys-Asn-Gly-Lys-Thr-
Cys-Asp-Cys-Pro-Arg-Asn-Pro-His-Lys-Gly-Pro-Ala-
Thr

**10.** A process according to claim 1 wherein the polypeptide obtained has the following amino acid sequence:

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Trp-Met-Asp-Asp-Tyr-Cys-Asn-Gly-Lys-Thr-
Cys-Asp-Cys-Pro-Arg-Asn-Pro-His-Lys-Gly-Pro-Ala-
Thr

**11.** A process according to claim 1, wherein the polypeptide obtained has the following amino acid sequence:

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Pro-Arg-
Gly-Asp-Phe-Leu-Asp-Asp-Tyr-Cys-Asn-Gly-Lys-Thr-
Cys-Asp-Cys-Pro-Arg-Asn-Pro-His-Lys-Gly-Pro-Ala-
Thr

**12.** A process according to claim 1, wherein the polypeptide obtained has the following aminoacid sequence:

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Asp-Leu-Asp-Asp-Tyr-Cys-Asn-Gly-Lys-Thr-
Cys-Asp-Cys-Pro-Arg-Asn-Pro-His-Lys-Gly-Pro-Ala-
Thr

**13.** A process according to claim 1, wherein the polypeptide obtained has the following amino acid sequence:

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Asp-Phe-Asp-Asp-Tyr-Cys-Asn-Gly-Lys-Thr-
Cys-Asp-Cys-Pro-Arg-Asn-Pro-His-Lys-Gly-Pro-Ala-
Thr

**14.** A process according to claim 1, wherein the polypeptide obtained has the following aminoacid sequence:

Glu-Cys-Glu-Ser-Gly-Pro-Cys-Cys-Arg-Asn-Cys-Lys-
Phe-Leu-Lys-Glu-Gly-Thr-Ile-Cys-Lys-Asn-Ala-Arg-
Gly-Asp-Phe-Leu-Asp-Asp-Tyr-Cys-Asn-Gly-Lys-Thr-
Cys-Asp-Cys-Pro-Arg-Asn-Pro-His-Lys-Gly-Pro-Ala-
Thr

**15.** A method for purifying a polypeptide of the following sequence:

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Asp-Met-Asp-Asp-Tyr-Cys-Asn-Gly-Lys-Thr-
Cys-Asp-Cys-Pro-Arg-Asn-Pro-His-Lys-Gly-Pro-Ala-
Thr

comprising
a) Dissolving lyophilized Echis carinatus venom in slightly basic solution;
b) Centrifuging the solution to obtain supernatant;
c) Loading supernatant onto a column; and
d) Determining fractions containing platelet aggregation inhibitory activity and concentrating under vacuum.

**Patentansprüche**
**Patentansprüche für folgende Vertragsstaaten : AT, BE, CH, LI, DE, FR, GB, IT, LU, NL, SE**

**1.** Polypeptid mit der folgenden Aminosäuresequenz

X-Cys-R-R-R-Arg-Gly-Asp-R-R-R-R-R-Cys-Y

wobei X H oder mindestens eine Aminosäure ist, Y OH oder mindestens eine Aminosäure ist und jedes R, die entweder gleich oder verschieden sind, irgendeine Aminosäure ist, mit der Ausnahme von Trigramin.

**2.** Polypeptid nach Anspruch 1 mit der folgenden Aminosäuresequenz

$H_2N$-(Ch)-Cys-R-R-R-Arg-Gly-Asp-R-R-R-R-R-Cys-(Cx)-H

wobei Ch mindestens eine Aminosäure bedeutet, Cx mindestens eine Aminosäure bedeutet und jedes R, die entweder gleich oder verschieden sind, eine Aminosäure bedeutet.

**3.** Im wesentlichen reines Polypeptid nach Anspruch 1, das "Echistatin" genannt wird und die folgende Aminosäuresequenz besitzt

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Asp-Met-Asp-Asp-Tyr-Cys-Asn-Gly-Lys-Thr-
Cys-Asp-Cys-Pro-Arg-Asn-Pro-His-Lys-Gly-Pro-Ala-
Thr

4. Im wesentlichen reines Polypeptid nach Anspruch 1, das "Bitistatin 1" genannt wird und die folgende Aminosäuresequenz besitzt

Ser-Pro-Pro-Val-Cys-Gly-Asn-Glu-Leu-Leu-Glu-Glu-
Gly-Glu-Glu-Cys-Asp-Cys-Gly-Ser-Pro-Ala-Asn-Cys-
Gln-Asp-Arg-Cys-Cys-Asn-Ala-Ala-Thr-Cys-Lys-Leu-
Thr-Pro-Gly-Ser-Gln-Cys-Asn-His-Gly-Glu-Cys-Cys-
Asp-Gln-Cys-Lys-Phe-Lys-Lys-Ala-Arg-Thr-Val-Cys-
Arg-Ile-Ala-Arg-Gly-Asp-Trp-Asn-Asp-Asp-Tyr-Cys-
Thr-Gly-Lys-Ser-Ser-Asp-Cys-Pro-Trp-Asn-His

5. Im wesentlichen reines Polypeptid nach Anspruch 1, das "Bitistatin 2" genannt wird und die folgende Aminosäuresequenz besitzt

Ser-Pro-Pro-Val-Cys-Gly-Asn-Lys-Ile-Leu-Glu-Gln-
Gly-Glu-Asp-Cys-Asp-Cys-Gly-Ser-Pro-Ala-Asn-Cys-
Gln-Asp-Gln-Cys-Cys-Asn-Ala-Ala-Thr-Cys-Lys-Leu-
Thr-Pro-Gly-Ser-Gln-Cys-Asn-His-Gly-Glu-Cys-Cys-
Asp-Gln-Asp-Lys-Phe-Lys-Lys-Ala-Arg-Thr-Val-Cys-
Arg-Ile-Ala-Arg-Gly-Asp-Trp-Asn-Asp-Asp-Tyr-Cys-
Thr-Gly-Lys-Ser-Ser-Asp-Cys-Pro-Trp-Asn-His

6. Im wesentlichen reines Polypeptid nach Anspruch 1, das "Bitistatin 3" genannt wird und die folgende Aminosäuresequenz besitzt

Val-Ser-Pro-Pro-Val-Cys-Gly-Asn-Lys-Ile-Leu-Glu-
Gln-Gly-Glu-Asp-Cys-Asp-Cys-Gly-Ser-Pro-Ala-Asn-
Cys-Gln-Asp-Gln-Cys-Cys-Asn-Ala-Ala-Thr-Cys-Lys-
Leu-Thr-Pro-Gly-Ser-Gln-Cys-Asn-His-Gly-Glu-Cys-
Cys-Asp-Gln-Cys-Lys-Phe-Lys-Lys-Ala-Arg-Thr-Val-
Cys-Arg-Ile-Ala-Arg-Gly-Asp-Trp-Asn-Asp-Asp-Tyr-
Cys-Thr-Gly-Lys-Ser-Ser-Asp-Cys-Pro-Trp-Asn-His

7. Im wesentlichen reines Polypeptid nach Anspruch 1, das "Eristostatin" genannt wird und die folgende Aminosäuresequenz besitzt

Gln-Glu-Glu-Pro-Cys-Ala-Thr-Gly-Pro-Cys-Cys-Arg-Arg-Cys-Lys-Phe-Lys-Arg-Ala-Gly-Lys-Val-Cys-Arg-Val-Ala-Arg-Gly-Asp-Trp-Asn-Asp-Asp-Tyr-Cys-Thr-Gly-Lys-Ser-Cys-Asp-Cys-Pro-Arg-Asn-Pro-Trp-Asn-His

8. Polypeptid nach Anspruch 1, das die folgende Aminosäuresequenz besitzt

Glu-Cys-Glu-Ser-Gly-Pro-Cys-Cys-Arg-Asn-Cys-Lys-Phe-Leu-Lys-Glu-Gly-Thr-Ile-Cys-Lys-Arg-Ala-Arg-Gly-Asp-Phe-Met-Asp-Asp-Tyr-Cys-Asn-Gly-Lys-Thr-Cys-Asp-Cys-Pro-Arg-Asn-Pro-His-Lys-Gly-Pro-Ala-Thr

9. Polypeptid nach Anspruch 1, das die folgende Aminosäuresequenz besitzt

Glu-Cys-Glu-Ser-Gly-Pro-Cys-Cys-Arg-Asn-Cys-Lys-Phe-Leu-Lys-Glu-Gly-Thr-Ile-Cys-Lys-Arg-Ala-Arg-Gly-Asp-Phe-Leu-Asp-Asp-Tyr-Cys-Asn-Gly-Lys-Thr-Cys-Asp-Cys-Pro-Arg-Asn-Pro-His-Lys-Gly-Pro-Ala-Thr

10. Polypeptid nach Anspruch 1, das die folgende Aminosäuresequenz besitzt

Glu-Cys-Glu-Ser-Gly-Pro-Cys-Cys-Arg-Asn-Cys-Lys-Phe-Leu-Lys-Glu-Gly-Thr-Ile-Cys-Lys-Arg-Ala-Arg-Gly-Asp-Trp-Met-Asp-Asp-Tyr-Cys-Asn-Gly-Lys-Thr-Cys-Asp-Cys-Pro-Arg-Asn-Pro-His-Lys-Gly-Pro-Ala-Thr

11. Polypeptid nach Anspruch 1, das die folgende Aminosäuresequenz besitzt

Glu-Cys-Glu-Ser-Gly-Pro-Cys-Cys-Arg-Asn-Cys-Lys-Phe-Leu-Lys-Glu-Gly-Thr-Ile-Cys-Lys-Arg-Pro-Arg-Gly-Asp-Phe-Leu-Asp-Asp-Tyr-Cys-Asn-Gly-Lys-Thr-Cys-Asp-Cys-Pro-Arg-Asn-Pro-His-Lys-Gly-Pro-Ala-Thr

**12.** Polypeptid nach Anspruch 1, das die folgende Aminosäuresequenz besitzt

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Asp-Leu-Asp-Asp-Tyr-Cys-Asn-Gly-Lys-Thr-
Cys-Asp-Cys-Pro-Arg-Asn-Pro-His-Lys-Gly-Pro-Ala-
Thr

**13.** Polypeptid nach Anspruch 1, das die folgende Aminosäuresequenz besitzt

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Asp-Phe-Asp-Asp-Tyr-Cys-Asn-Gly-Lys-Thr-
Cys-Asp-Cys-Pro-Arg-Asn-Pro-His-Lys-Gly-Pro-Ala-
Thr

**14.** Polypeptid nach Anspruch 1, das die folgende Aminosäuresequenz besitzt

Glu-Cys-Glu-Ser-Gly-Pro-Cys-Cys-Arg-Asn-Cys-Lys-
Phe-Leu-Lys-Glu-Gly-Thr-Ile-Cys-Lys-Asn-Ala-Arg-
Gly-Asp-Phe-Leu-Asp-Asp-Tyr-Cys-Asn-Gly-Lys-Thr-
Cys-Asp-Cys-Pro-Arg-Asn-Pro-His-Lys-Gly-Pro-Ala-
Thr

**15.** Verfahren zur Hemmung der Fibrinogen-Bindung an Blutplättchen von Säugetieren oder der Fibrinogen-induzierten Aggregation von Blutplättchen von Säugetieren, umfassend das Inkubieren von Blutplättchen von Säugetieren mit einem Polypeptid nach Anspruch 1.

**16.** Verwendung eines Polypeptids nach Anspruch 1 zur Herstellung eines Arzneimittels, das geeignet ist zur Hemmung der Aggregation von Blutplättchen bei einem Säugetier.

**17.** Verfahren zur Reinigung eines Polypeptids mit der folgenden Sequenz

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Asp-Met-Asp-Asp-Tyr-Cys-Asn-Gly-Lys-Thr-
Cys-Asp-Cys-Pro-Arg-Asn-Pro-His-Lys-Gly-Pro-Ala-
Thr

umfassend

(a) Auflösen von lyophilisiertem Echis carinatus Gift in leicht basischer Lösung;
(b) Zentrifugieren der Lösung, um einen Überstand zu erhalten;

(c) Aufbringen der Lösung auf eine Säule und
(d) Bestimmung von Fraktionen, die die Blutplättchen-Aggregation hemmende Wirkung besitzen und Konzentrieren unter Vakuum.

**18.** DNA-Molekül, umfassend eine Rekombinations DNA, die ein Polypeptid mit der folgenden Aminosäuresequenz kodiert

$H_2N$-(Ch)-Cys-R-R-R-Arg-Gly-Asp-R-R-R-R-R-Cys-(Cx)-H

wobei Ch mindestens eine Aminosäure bedeutet, Cx mindestens eine Aminosäure bedeutet und jedes R, die entweder gleich oder verschieden sind, eine Aminosäure bedeutet, wobei dieses Peptid die Blutplättchen-Aggregation hemmt.

**19.** DNA-Molekül nach Anspruch 18, wobei die Aminosäuresequenz wie folgt ist:

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Phe-Leu-Asp-Asp-Tyr-Cys-Asn-Gly-Lys-Thr-
Cys-Asp-Cys-Pro-Arg-Asn-Pro-His-Lys-Gly-Pro-Ala-
Thr

**20.** Reproduzierbarer mikrobiologischer Expressions-Vektor, umfassend eine Promotor/Operator-Sequenz, die in der Lage ist, heterologe Proteine in einem Mikroorganismus zu exprimieren, gefolgt von DNA-Kodierung eines Blutplättchen-Aggregations-Hemmers mit der Aminosäuresequenz

$H_2N$-(Ch)-Cys-R-R-R-Arg-Gly-Asp-R-R-R-R-R-Cys-(Cx)-H

wobei Ch mindestens eine Aminosäure bedeutet, Cx mindestens eine Aminosäure bedeutet und jedes R, die entweder gleich oder verschieden sind, eine Aminosäure bedeutet, wobei die Transkription der DNA in einem Transformations-Mikroorganismus unter Kontrolle des Promotor/Operators erfolgt.

**21.** Expressionsvektor nach Anspruch 20, wobei die Aminosäuresequenz die folgende Aminosäuresequenz besitzt:

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Asp-Leu-Asp-Asp-Tyr-Cys-Asn-Gly-Lys-Thr-
Cys-Asp-Cys-Pro-Arg-Asn-Pro-His-Lys-Gly-Pro-Ala-
Thr

**22.** Lebensfähgige Zellkultur, umfassend mit dem Expressions-Vektor nach Anspruch 20 transformierte Zellen.

**23.** Verfahren zur Herstellung eines Cystein-reichen Polypeptids, enthaltend die folgende Sequenz:

$H_2N$-(Ch)-Cys-R-R-R-Arg-Gly-Asp-R-R-R-R-R-Cys-(Cx)-H

wobei Ch mindestens eine Aminosäure bedeutet, Cx mindestens eine Aminosäure bedeutet und jedes R, die entweder gleich oder verschieden sind, eine Aminosäure bedeutet, wobei

(a) die C-terminal erste Aminosäure der Sequenz an ein festes vernetztes Polystyrolharz gebunden wird unter Bildung eines Amino-acyl-Polymers, die die C-terminal erste Aminosäure blockierende Gruppe von dem Amino-acyl-Polymer entfernt wird, die in der Sequenz nächste zweite Aminosäure an den C-terminalen Aminosäure-Rest gekoppelt wird, folgende Aminosäuren schrittweise angekoppelt werden unter Bildung der Polypeptid-Sequenz und die N-terminale Boc-Gruppe, die die N-terminale Aminosäure schützt, entfernt wird;

(b) die Polypeptid-Sequenz mit HF und einem oder mehreren Thiohaltigen Abfangmitteln behandelt wird;

(c) die behandelte Sequenz gewaschen und von der Säule in ein großes Volumen verdünnte Essigsäure überführt wird, um eine unerwünschte Vernetzung möglichst gering zu halten und

(d) die aus Stufe (c) stammende Lösung unter Rühren auf einen pH-Wert von etwa 8 eingestellt wird.

**Patentansprüch für folgende Vertragsstaaten : ES, GR**

**1.** Verfahren zur Herstellung eines Cystein-reichen Polypeptids mit der folgenden Aminosäuresequenz

X-Cys-R-R-R-Arg-Gly-Asp-R-R-R-R-R-Cys-Y

wobei

X H oder mindestens eine Aminosäure ist,

Y OH oder mindestens eine Aminosäure ist und

jedes R, die entweder gleich oder verschieden sind, irgendeine Aminosäure ist,

mit der Ausnahme von Trigramin,

umfassend

a) Reinigen des Polypeptids aus dem Gift von verschiedenen Vipern, wie Echis carinatus, Agkistrodon piscivorus, Bitis arientans und Eristocophis macmohonii, oder wahlweise

b) Exprimieren des Gens, das das gewünschte Polypeptid kodiert in einen geeigneten Wirt durch Rekombinations-DNA-Technologie oder wahlweise

c) chemisches Synthetisieren des gewünschten Polypeptids durch eine Synthese nach Merrifield oder eine Synthese nach Houghten.

**2.** Verfahren nach Anspruch 1, wobei das Polypeptid die folgende Sequenz umfaßt

$H_2N$-(Ch)-Cys-R-R-R-Arg-Gly-Asp-R-R-R-R-R-Cys-(Cx)-H

wobei

Ch mindestens eine Aminosäure bedeutet,

Cx mindestens eine Aminosäure bedeutet und

jedes R, die entweder gleich oder verschieden sind, eine Aminosäure bedeutet,

wobei das Verfahren dadurch gekennzeichnet ist, daß.

(a) die C-terminal erste Aminosäure der Sequenz an ein festes vernetztes Polystyrolharz gebunden wird unter Bildung eines Amino-acyl-Polymers, die die C-terminal erste Aminosäure blockierende Gruppe von dem Amino-acyl-Polymer entfernt wird, die in der Sequenz nächste zweite Aminosäure an den C-terminalen Aminosäure-Rest gekoppelt wird, folgende Aminosäuren schrittweise angekoppelt werden unter Bildung der Polypeptid-Sequenz und die N-terminale Boc-Gruppe, die die N-terminale Aminosäure schützt, entfernt wird;

(b) die Polypeptid-Sequenz mit HF und einem oder mehreren Thiohaltigen Abfangmitteln behandelt wird;

(c) die behandelte Sequenz gewaschen und von der Säule in ein großes Volumen verdünnte Essigsäure überführt wird, um eine unerwünschte Vernetzung möglichst gering zu halten und

(d) die aus Stufe (c) stammende Lösung unter Rühren auf einen pH-Wert von etwa 8 eingestellt wird.

**3.** Verfahren nach Anspruch 1, wobei das erhaltene Polypeptid "Echistatin" genannt wird und die folgende Aminosäuresequenz besitzt

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Asp-Met-Asp-Asp-Tyr-Cys-Asn-Gly-Lys-Thr-
Cys-Asp-Cys-Pro-Arg-Asn-Pro-His-Lys-Gly-Pro-Ala-
Thr

4. Verfahren nach Anspruch 1, wobei das erhaltene Polypeptid "Bitistatin 1" genannt wird und die folgende Aminosäuresequenz besitzt

Ser-Pro-Pro-Val-Cys-Gly-Asn-Glu-Leu-Leu-Glu-Glu-
Gly-Glu-Glu-Cys-Asp-Cys-Gly-Ser-Pro-Ala-Asn-Cys-
Gln-Asp-Arg-Cys-Cys-Asn-Ala-Ala-Thr-Cys-Lys-Leu-
Thr-Pro-Gly-Ser-Gln-Cys-Asn-His-Gly-Glu-Cys-Cys-
Asp-Gln-Cys-Lys-Phe-Lys-Lys-Ala-Arg-Thr-Val-Cys-
Arg-Ile-Ala-Arg-Gly-Asp-Trp-Asn-Asp-Asp-Tyr-Cys-
Thr-Gly-Lys-Ser-Ser-Asp-Cys-Pro-Trp-Asn-His

5. Verfahren nach Anspruch 1, wobei das erhaltene Polypeptid "Bitistatin 2" genannt wird und die folgende Aminosäuresequenz besitzt

Ser-Pro-Pro-Val-Cys-Gly-Asn-Lys-Ile-Leu-Glu-Gln-
Gly-Glu-Asp-Cys-Asp-Cys-Gly-Ser-Pro-Ala-Asn-Cys-
Gln-Asp-Gln-Cys-Cys-Asn-Ala-Ala-Thr-Cys-Lys-Leu-
Thr-Pro-Gly-Ser-Gln-Cys-Asn-His-Gly-Glu-Cys-Cys-
Asp-Gln-Asp-Lys-Phe-Lys-Lys-Ala-Arg-Thr-Val-Cys-
Arg-Ile-Ala-Arg-Gly-Asp-Trp-Asn-Asp-Asp-Tyr-Cys-
Thr-Gly-Lys-Ser-Ser-Asp-Cys-Pro-Trp-Asn-His

6. Verfahren nach Anspruch 1, wobei das erhaltene Polypeptid "Bitistatin 3" genannt wird und die folgende Aminosäuresequenz besitzt

Val-Ser-Pro-Pro-Val-Cys-Gly-Asn-Lys-Ile-Leu-Glu-
Gln-Gly-Glu-Asp-Cys-Asp-Cys-Gly-Ser-Pro-Ala-Asn-
Cys-Gln-Asp-Gln-Cys-Cys-Asn-Ala-Ala-Thr-Cys-Lys-
Leu-Thr-Pro-Gly-Ser-Gln-Cys-Asn-His-Gly-Glu-Cys-
Cys-Asp-Gln-Cys-Lys-Phe-Lys-Lys-Ala-Arg-Thr-Val-
Cys-Arg-Ile-Ala-Arg-Gly-Asp-Trp-Asn-Asp-Asp-Tyr-
Cys-Thr-Gly-Lys-Ser-Ser-Asp-Cys-Pro-Trp-Asn-His

7. Verfahren nach Anspruch 1, wobei das erhaltene Polypeptid "Eristostatin" genannt wird und die folgende Aminosäuresequenz besitzt

Gln-Glu-Glu-Pro-Cys-Ala-Thr-Gly-Pro-Cys-Cys-Arg-
Arg-Cys-Lys-Phe-Lys-Arg-Ala-Gly-Lys-Val-Cys-Arg-
Val-Ala-Arg-Gly-Asp-Trp-Asn-Asp-Asp-Tyr-Cys-Thr-
Gly-Lys-Ser-Cys-Asp-Cys-Pro-Arg-Asn-Pro-Trp-Asn-
His

8. Verfahren nach Anspruch 1, wobei das erhaltene Polypeptid die folgende Aminosäuresequenz besitzt

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Phe-Met-Asp-Asp-Tyr-Cys-Asn-Gly-Lys-Thr-
Cys-Asp-Cys-Pro-Arg-Asn-Pro-His-Lys-Gly-Pro-Ala-
Thr

9. Verfahren nach Anspruch 1, wobei das erhaltene Polypeptid die folgende Aminosäuresequenz besitzt

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Phe-Leu-Asp-Asp-Tyr-Cys-Asn-Gly-Lys-Thr-
Cys-Asp-Cys-Pro-Arg-Asn-Pro-His-Lys-Gly-Pro-Ala-
Thr

10. Verfahren nach Anspruch 1, wobei das erhaltene Polypeptid die folgende Aminosäuresequenz besitzt

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Trp-Met-Asp-Asp-Tyr-Cys-Asn-Gly-Lys-Thr-
Cys-Asp-Cys-Pro-Arg-Asn-Pro-His-Lys-Gly-Pro-Ala-
Thr

**11.** Verfahren nach Anspruch 1, wobei das erhaltene Polypeptid die folgende Aminosäuresequenz besitzt

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Pro-Arg-
Gly-Asp-Phe-Leu-Asp-Asp-Tyr-Cys-Asn-Gly-Lys-Thr-
Cys-Asp-Cys-Pro-Arg-Asn-Pro-His-Lys-Gly-Pro-Ala-
Thr

**12.** Verfahren nach Anspruch 1, wobei das erhaltene Polypeptid die folgende Aminosäuresequenz besitzt

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Asp-Leu-Asp-Asp-Tyr-Cys-Asn-Gly-Lys-Thr-
Cys-Asp-Cys-Pro-Arg-Asn-Pro-His-Lys-Gly-Pro-Ala-
Thr

**13.** Verfahren nach Anspruch 1, wobei das erhaltene Polypeptid die folgende Aminosäuresequenz besitzt

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Asp-Phe-Asp-Asp-Tyr-Cys-Asn-Gly-Lys-Thr-
Cys-Asp-Cys-Pro-Arg-Asn-Pro-His-Lys-Gly-Pro-Ala-
Thr

**14.** Verfahren nach Anspruch 1, wobei das erhaltene Polypeptid die folgende Aminosäuresequenz besitzt

Glu-Cys-Glu-Ser-Gly-Pro-Cys-Cys-Arg-Asn-Cys-Lys-
Phe-Leu-Lys-Glu-Gly-Thr-Ile-Cys-Lys-Asn-Ala-Arg-
Gly-Asp-Phe-Leu-Asp-Asp-Tyr-Cys-Asn-Gly-Lys-Thr-
Cys-Asp-Cys-Pro-Arg-Asn-Pro-His-Lys-Gly-Pro-Ala-
Thr

**15.** Verfahren zum Reinigen eines Polypeptids mit der folgenden Sequenz

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Asp-Met-Asp-Asp-Tyr-Cys-Asn-Gly-Lys-Thr-
Cys-Asp-Cys-Pro-Arg-Asn-Pro-His-Lys-Gly-Pro-Ala-
Thr

umfassend

(a) Auflösen von lyophilisiertem Echis carinatus Gift in leicht basischer Lösung;
(b) Zentrifugieren der Lösung, um einen Überstand zu erhalten;
(c) Aufbringen der Lösung auf eine Säule und
(d) Bestimmung von Fraktionen, die die Blutplättchen-Aggregation hemmende Wirkung besitzen und Konzentrieren unter Vakuum.

**Revendications**

**Revendications pour les Etats contractants suivants : AT, BE, CH, LI, DE, FR, GB, IT, LU, NL, SE**

1. Polypeptide ayant la séquence d'acides aminés suivante:

X-Cys-R-R-R-Arg-Gly-Asp-R-R-R-R-R-Cys-Y

où X est H ou au moins un acide aminé; Y est OH ou au moins un acide aminé; et chacun des R, soit identiques soit différents, est un acide aminé quelconque, à l'exception de la trigramine.

2. Polypeptides selon la revendication 1, ayant la séquence d'acides aminés suivante:

$H_2N$-(Ch)-Cys-R-R-R-Arg-Gly-Asp-R-R-R-R-R-Cys-(Cx)-H

où Ch représente au moins un acide aminé; Cx représente au moins un acide aminé; et chacun des groupes R, qui sont identiques ou différents, représente un acide aminé.

3. Polypeptide pratiquement pur selon la revendication 1 dénommé "échistatine", ayant la séquence d'acides aminés suivante:

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Asp-Met-Asp-Asp-Tyr-Cys-Asn-Gly-Lys-Thr-
Cys-Asp-Cys-Pro-Arg-Asn-Pro-His-Lys-Gly-Pro-Ala-
Thr

4. Polypeptide pratiquement pur selon la revendication 1 dénommé "bitistatine 1", ayant la séquence suivante:

Ser-Pro-Pro-Val-Cys-Gly-Asn-Glu-Leu-Leu-Glu-Glu-
Gly-Glu-Glu-Cys-Asp-Cys-Gly-Ser-Pro-Ala-Asn-Cys-
Gln-Asp-Arg-Cys-Cys-Asn-Ala-Ala-Thr-Cys-Lys-Leu-
Thr-Pro-Gly-Ser-Gln-Cys-Asn-His-Gly-Glu-Cys-Cys-
Asp-Gln-Cys-Lys-Phe-Lys-Lys-Ala-Arg-Thr-Val-Cys-
Arg-Ile-Ala-Arg-Gly-Asp-Trp-Asn-Asp-Asp-Tyr-Cys-
Thr-Gly-Lys-Ser-Ser-Asp-Cys-Pro-Trp-Asn-His

5. Polypeptide pratiquement pur selon la revendication 1 dénommé "bitistatine 2", ayant la séquence suivante:

Ser-Pro-Pro-Val-Cys-Gly-Asn-Lys-Ile-Leu-Glu-Gln-Gly-Glu-Asp-Cys-Asp-Cys-Gly-Ser-Pro-Ala-Asn-Cys-Gln-Asp-Gln-Cys-Cys-Asn-Ala-Ala-Thr-Cys-Lys-Leu-Thr-Pro-Gly-Ser-Gln-Cys-Asn-His-Gly-Glu-Cys-Cys-Asp-Gln-Asp-Lys-Phe-Lys-Lys-Ala-Arg-Thr-Val-Cys-Arg-Ile-Ala-Arg-Gly-Asp-Trp-Asn-Asp-Asp-Tyr-Cys-Thr-Gly-Lys-Ser-Ser-Asp-Cys-Pro-Trp-Asn-His

**6.** Polypeptide pratiquement pur selon la revendication 1 dénommé "bitistatine 3", ayant la séquence suivante:

Val-Ser-Pro-Pro-Val-Cys-Gly-Asn-Lys-Ile-Leu-Glu-Gln-Gly-Glu-Asp-Cys-Asp-Cys-Gly-Ser-Pro-Ala-Asn-Cys-Gln-Asp-Gln-Cys-Cys-Asn-Ala-Ala-Thr-Cys-Lys-Leu-Thr-Pro-Gly-Ser-Gln-Cys-Asn-His-Gly-Glu-Cys-Cys-Asp-Gln-Cys-Lys-Phe-Lys-Lys-Ala-Arg-Thr-Val-Cys-Arg-Ile-Ala-Arg-Gly-Asp-Trp-Asn-Asp-Asp-Tyr-Cys-Thr-Gly-Lys-Ser-Ser-Asp-Cys-Pro-Trp-Asn-His

**7.** Polypeptide pratiquement pur selon la revendication 1 dénommé "éristostatine", ayant la séquence d'acides aminés suivante:

Gln-Glu-Glu-Pro-Cys-Ala-Thr-Gly-Pro-Cys-Cys-Arg-Arg-Cys-Lys-Phe-Lys-Arg-Ala-Gly-Lys-Val-Cys-Arg-Val-Ala-Arg-Gly-Asp-Trp-Asn-Asp-Asp-Tyr-Cys-Thr-Gly-Lys-Ser-Cys-Asp-Cys-Pro-Arg-Asn-Pro-Trp-Asn-His

**8.** Polypeptide selon la revendication 1 ayant la séquence d'acides aminés suivante:

Glu-Cys-Glu-Ser-Gly-Pro-Cys-Cys-Arg-Asn-Cys-Lys-Phe-Leu-Lys-Glu-Gly-Thr-Ile-Cys-Lys-Arg-Ala-Arg-Gly-Asp-Phe-Met-Asp-Asp-Tyr-Cys-Asn-Gly-Lys-Thr-Cys-Asp-Cys-Pro-Arg-Asn-Pro-His-Lys-Gly-Pro-Ala-Thr

**9.** Polypeptide selon la revendication 1 ayant la séquence d'acides aminés suivante:

Glu-Cys-Glu-Ser-Gly-Pro-Cys-Cys-Arg-Asn-Cys-Lys-Phe-Leu-Lys-Glu-Gly-Thr-Ile-Cys-Lys-Arg-Ala-Arg-Gly-Asp-Phe-Leu-Asp-Asp-Tyr-Cys-Asn-Gly-Lys-Thr-Cys-Asp-Cys-Pro-Arg-Asn-Pro-His-Lys-Gly-Pro-Ala-Thr

**10.** Polypeptide selon la revendication 1 ayant la séquence d'acides aminés suivante:

Glu-Cys-Glu-Ser-Gly-Pro-Cys-Cys-Arg-Asn-Cys-Lys-Phe-Leu-Lys-Glu-Gly-Thr-Ile-Cys-Lys-Arg-Ala-Arg-Gly-Asp-Trp-Met-Asp-Asp-Tyr-Cys-Asn-Gly-Lys-Thr-Cys-Asp-Cys-Pro-Arg-Asn-Pro-His-Lys-Gly-Pro-Ala-Thr

**11.** Polypeptide selon la revendication 1 ayant la séquence d'acides aminés suivante:

Glu-Cys-Glu-Ser-Gly-Pro-Cys-Cys-Arg-Asn-Cys-Lys-Phe-Leu-Lys-Glu-Gly-Thr-Ile-Cys-Lys-Arg-Pro-Arg-Gly-Asp-Phe-Leu-Asp-Asp-Tyr-Cys-Asn-Gly-Lys-Thr-Cys-Asp-Cys-Pro-Arg-Asn-Pro-His-Lys-Gly-Pro-Ala-Thr

**12.** Polypeptide selon la revendication 1 ayant la séquence d'acides aminés suivante:

Glu-Cys-Glu-Ser-Gly-Pro-Cys-Cys-Arg-Asn-Cys-Lys-Phe-Leu-Lys-Glu-Gly-Thr-Ile-Cys-Lys-Arg-Ala-Arg-Gly-Asp-Asp-Leu-Asp-Asp-Tyr-Cys-Asn-Gly-Lys-Thr-Cys-Asp-Cys-Pro-Arg-Asn-Pro-His-Lys-Gly-Pro-Ala-Thr

**13.** Polypeptide selon la revendication 1 ayant la séquence d'acides aminés suivante:

Glu-Cys-Glu-Ser-Gly-Pro-Cys-Cys-Arg-Asn-Cys-Lys-Phe-Leu-Lys-Glu-Gly-Thr-Ile-Cys-Lys-Arg-Ala-Arg-Gly-Asp-Asp-Phe-Asp-Asp-Tyr-Cys-Asn-Gly-Lys-Thr-Cys-Asp-Cys-Pro-Arg-Asn-Pro-His-Lys-Gly-Pro-Ala-Thr

**14.** Polypeptide selon la revendication 1 ayant la séquence d'acides aminés suivante:

Glu-Cys-Glu-Ser-Gly-Pro-Cys-Cys-Arg-Asn-Cys-Lys-Phe-Leu-Lys-Glu-Gly-Thr-Ile-Cys-Lys-Asn-Ala-Arg-Gly-Asp-Phe-Leu-Asp-Asp-Tyr-Cys-Asn-Gly-Lys-Thr-Cys-Asp-Cys-Pro-Arg-Asn-Pro-His-Lys-Gly-Pro-Ala-Thr

**15.** Procédé pour l'inhibition de la fixation du fibrinogène à des plaquettes de mammifères ou de l'agrégation induite par le fibrinogène de plaquettes de mammifères, comprenant l'incubation de plaquettes de mammifères avec un polypeptide selon la revendication 1.

**16.** Utilisation d'un polypeptide selon la revendication 1 pour la fabrication d'un médicament utile pour l'inhibition de l'agrégation plaquettaire chez un mammifère.

**17.** Procédé pour la purification d'un polypeptide ayant la séquence suivante:

Glu-Cys-Glu-Ser-Gly-Pro-Cys-Cys-Arg-Asn-Cys-Lys-Phe-Leu-Lys-Glu-Gly-Thr-Ile-Cys-Lys-Arg-Ala-Arg-Gly-Asp-Asp-Met-Asp-Asp-Tyr-Cys-Asn-Gly-Lys-Thr-Cys-Asp-Cys-Pro-Arg-Asn-Pro-His-Lys-Gly-Pro-Ala-Thr

comprenant

(a) la dissolution de venin de <u>Echis</u> <u>carinatus</u> lyophilisé dans une solution légèrement basique;
(b) la centrifugation de la solution pour obtenir un surnageant;
(c) le chargement du surnageant sur une colonne et
(d) la détermination des fractions contenant une activité d'inhibition de l'agrégation plaquettaire et la concentration sous vide.

**18.** Molécule d'ADN comprenant un ADN recombiné codant pour un polypeptide ayant la séquence d'acides aminés suivante:

$H_2$-(Ch)-Cys-R-R-R-Arg-Gly-Asp-R-R-R-R-R-Cys-(Cx)-H

où Ch représente au moins un acide aminé; Cx représente au moins un acide aminé; et chacun des groupes R, qui sont identiques ou différents, représente un acide aminé, ledit polypeptide inhibant l'agrégation plaquettaire.

**19.** Molécule d'ADN selon la revendication 18, dans laquelle la séquence d'acides aminés est la suivante:

Glu-Cys-Glu-Ser-Gly-Pro-Cys-Cys-Arg-Asn-Cys-Lys-Phe-Leu-Lys-Glu-Gly-Thr-Ile-Cys-Lys-Arg-Ala-Arg-Gly-Asp-Phe-Leu-Asp-Asp-Tyr-Cys-Asn-Gly-Lys-Thr-Cys-Asp-Cys-Pro-Arg-Asn-Pro-His-Lys-Gly-Pro-Ala-Thr

**20.** Vecteur d'expression microbien réplicable, comprenant une séquence de promoteur-opérateur apte à exprimer des protéines hétérologues dans un microorganisme, suivie par un ADN codant pour un inhibiteur d'agrégation plaquettaire ayant une séquence d'acides aminés

$H_2N$-(Ch)-Cys-R-R-R-Arg-Gly-Asp-R-R-R-R-R-Cys-(Cx)-H

où Ch représente au moins un acide aminé; Cx représente au moins un acide aminé; et chacun des groupes R, qui sont identiques ou différents, représente un acide aminé, tandis que la transcription du dit ADN dans un microorganisme transformant est sous le contrôle du dit promoteur-opérateur.

**21.** Vecteur d'expression selon la revendication 20, dans lequel la séquence d'acides aminés a la séquence d'acides aminés suivante:

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Asp-Leu-Asp-Asp-Tyr-Cys-Asn-Gly-Lys-Thr-
Cys-Asp-Cys-Pro-Arg-Asn-Pro-His-Lys-Gly-Pro-Ala-
Thr

**22.** Culture de cellules viables comprenant des cellules transformées avec le vecteur d'expression selon la revendication 21.

**23.** Procédé pour la préparation d'un polypeptide riche en cystéine contenant la séquence suivante:

$H_2N$-(Ch)-Cys-R-R-R-Arg-Gly-Asp-R-R-R-R-R-Cys-(Cx)-H

où Ch représente au moins un acide aminé; Cx représente au moins un acide aminé; et chacun des groupes R, qui sont identiques ou différents, représente un acide aminé, dans lequel:
(a) le premier acide aminé C-terminal de la séquence est fixé à une résine de polystyrène solide réticulée pour former un polymère d'amino acyle, le groupe bloquant le premier acide aminé C-terminal est éliminé du polymère d'amino acyle, le second acide aminé qui suit dans l'ordre de la séquence est couplé au résidu d'acide aminé C-terminal, les acides aminés suivants sont couplés par étapes pour former la séquence polypeptidique, et le groupe Boc N-terminal protégeant l'acide aminé N-terminal est éliminé;
(b) la séquence polypeptidique est traitée avec HF et un ou plusieurs piégeurs conteant des groupes thio;
(c) la séquence traitée est lavée et transférée de la colonne dans un grand volume d'acide acétique dilué pour minimiser toute réticulation indésirable; et
(d) la solution résultante de l'étape (c) est ajustée à un pH d'environ 8,0 sous agitation.

**Revendications pour les Etats contractants suivants : ES, GR**

**1.** Procédé pour la préparation d'un polypeptide riche en cystéine ayant la séquence d'acides aminés suivante:

X-Cys-R-R-R-Arg-Gly-Asp-R-R-R-R-R-Cys-Y

où
X est H ou au moins un acide aminé;
Y est H ou au moins un acide aminé;
et
chacun des groupes R, qui sont identiques ou différents, est un acide aminé quelconque;
à l'exception de la trigramine;
comprenant:

a) la purification du polypeptide du venin de diverses vipères, telles que Echis carinatus, Agkistrodon piscivorus, Bitis arietans et Eristocophis macmahonii; ou en variante
b) l'expression du gène qui code pour le polypeptide désiré, dans un hôte approprié par une technologie à l'ADN recombiné ou génie génétique; ou en variante
c) la synthèse chimique du polypeptide désiré par synthèse de Merrifield ou par synthèse de Houghten.

**2.** Procédé selon la revendication 1, dans lequel le polypeptide comprend la séquence suivante

$H_2N$-(Ch)-Cys-R-R-R-Arg-Gly-Asp-R-R-R-R-R-Cys-(Cx)-H

où
Ch représente au moins un acide aminé;
Cx représente au moins un acide aminé;
chacun des groupes R, qui sont identiques ou différents, représente un acide aminé;
le dit procédé étant caractérisé en ce que:
(a) le premier acide aminé C-terminal de la séquence est fixé à une résine de polystyrène solide réticulée pour former un polymère d'amino acyle, le groupe bloquant le premier acide aminé C-terminal est éliminé du polymère d'amino acyle, le second acide aminé qui suit dans l'ordre de la séquence est couplé au résidu d'acide aminé C-terminal, les acides aminés suivants sont couplés par étapes pour former la séquence polypeptidique, et le groupe Boc N-terminal protégeant l'acide aminé N-terminal est éliminé;
(b) la séquence polypeptidique est traitée avec HF et un ou plusieurs piégeurs conetant des groupes thio;
(c) la séquence traitée est lavée et transférée de la colonne dans un grand volume d'acide acétique dilué pour minimiser toute réticulation indésirable; et
(d) la solution résultante de l'étape (c) est ajustée à un pH d'environ 8,0 sous agitation.

**3.** Procédé selon la revendication 1, dans lequel le polypeptide obtenu est dénommé "échistatine" et a la séquence d'acides aminés suivante:

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Asp-Met-Asp-Asp-Tyr-Cys-Asn-Gly-Lys-Thr-
Cys-Asp-Cys-Pro-Arg-Asn-Pro-His-Lys-Gly-Pro-Ala-
Thr

**4.** Procédé selon la revendication 1, dans lequel le polypeptide obtenu est dénommé "bitistatine 1" et a la séquence d'acides aminés suivante:

Ser-Pro-Pro-Val-Cys-Gly-Asn-Glu-Leu-Leu-Glu-Glu-
Gly-Glu-Glu-Cys-Asp-Cys-Gly-Ser-Pro-Ala-Asn-Cys-
Gln-Asp-Arg-Cys-Cys-Asn-Ala-Ala-Thr-Cys-Lys-Leu-

Thr-Pro-Gly-Ser-Gln-Cys-Asn-His-Gly-Glu-Cys-Cys-
Asp-Gln-Cys-Lys-Phe-Lys-Lys-Ala-Arg-Thr-Val-Cys-
Arg-Ile-Ala-Arg-Gly-Asp-Trp-Asn-Asp-Asp-Tyr-Cys-
Thr-Gly-Lys-Ser-Ser-Asp-Cys-Pro-Trp-Asn-His

5. Procédé selon la revendication 1, dans lequel le polypeptide obtenu est dénommé "bitistatine 2" et a la séquence d'acides aminés suivante:

Ser-Pro-Pro-Val-Cys-Gly-Asn-Lys-Ile-Leu-Glu-Gln-
Gly-Glu-Asp-Cys-Asp-Cys-Gly-Ser-Pro-Ala-Asn-Cys-
Gln-Asp-Gln-Cys-Cys-Asn-Ala-Ala-Thr-Cys-Lys-Leu-
Thr-Pro-Gly-Ser-Gln-Cys-Asn-His-Gly-Glu-Cys-Cys-
Asp-Gln-Asp-Lys-Phe-Lys-Lys-Ala-Arg-Thr-Val-Cys-
Arg-Ile-Ala-Arg-Gly-Asp-Trp-Asn-Asp-Asp-Tyr-Cys-
Thr-Gly-Lys-Ser-Ser-Asp-Cys-Pro-Trp-Asn-His

6. Procédé selon la revendication 1, dans lequel le polypeptide obtenu est dénommé "bitistatine 3" et a la séquence d'acides aminés suivante:

Val-Ser-Pro-Pro-Val-Cys-Gly-Asn-Lys-Ile-Leu-Glu-
Gln-Gly-Glu-Asp-Cys-Asp-Cys-Gly-Ser-Pro-Ala-Asn-
Cys-Gln-Asp-Gln-Cys-Cys-Asn-Ala-Ala-Thr-Cys-Lys-
Leu-Thr-Pro-Gly-Ser-Gln-Cys-Asn-His-Gly-Glu-Cys-
Cys-Asp-Gln-Cys-Lys-Phe-Lys-Lys-Ala-Arg-Thr-Val-
Cys-Arg-Ile-Ala-Arg-Gly-Asp-Trp-Asn-Asp-Asp-Tyr-
Cys-Thr-Gly-Lys-Ser-Ser-Asp-Cys-Pro-Trp-Asn-His

7. Procédé selon la revendication 1, dans lequel le polypeptide obtenu est dénommé "éristostatine" et a la séquence d'acides aminés suivante:

Gln-Glu-Glu-Pro-Cys-Ala-Thr-Gly-Pro-Cys-Cys-Arg-
Arg-Cys-Lys-Phe-Lys-Arg-Ala-Gly-Lys-Val-Cys-Arg-
Val-Ala-Arg-Gly-Asp-Trp-Asn-Asp-Asp-Tyr-Cys-Thr-
Gly-Lys-Ser-Cys-Asp-Cys-Pro-Arg-Asn-Pro-Trp-Asn-
His

8. Procédé selon la revendication 1, dans lequel le polypeptide obtenu a la séquence d'acides aminés suivante:

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Phe-Met-Asp-Asp-Tyr-Cys-Asn-Gly-Lys-Thr-
Cys-Asp-Cys-Pro-Arg-Asn-Pro-His-Lys-Gly-Pro-Ala-
Thr

**9.** Procédé selon la revendication 1, dans lequel le polypeptide obtenu a la séquence d'acides aminés suivante:

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Phe-Leu-Asp-Asp-Tyr-Cys-Asn-Gly-Lys-Thr-
Cys-Asp-Cys-Pro-Arg-Asn-Pro-His-Lys-Gly-Pro-Ala-
Thr

**10.** Procédé selon la revendication 1, dans lequel le polypeptide obtenu a la séquence d'acides aminés suivante:

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Trp-Met-Asp-Asp-Tyr-Cys-Asn-Gly-Lys-Thr-
Cys-Asp-Cys-Pro-Arg-Asn-Pro-His-Lys-Gly-Pro-Ala-
Thr

**11.** Procédé selon la revendication 1, dans lequel le polypeptide obtenu a la séquence d'acides aminés suivante:

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Pro-Arg-
Gly-Asp-Phe-Leu-Asp-Asp-Tyr-Cys-Asn-Gly-Lys-Thr-
Cys-Asp-Cys-Pro-Arg-Asn-Pro-His-Lys-Gly-Pro-Ala-
Thr

**12.** Procédé selon la revendication 1, dans lequel le polypeptide obtenu a la séquence d'acides aminés suivante:

Glu-Cys-Glu-Ser-Gly-Pro-Cys-Cys-Arg-Asn-Cys-Lys-
Phe-Leu-Lys-Glu-Gly-Thr-Ile-Cys-Lys-Arg-Ala-Arg-
Gly-Asp-Asp-Leu-Asp-Asp-Tyr-Cys-Asn-Gly-Lys-Thr-
Cys-Asp-Cys-Pro-Arg-Asn-Pro-His-Lys-Gly-Pro-Ala-
Thr

**13.** Procédé selon la revendication 1, dans lequel le polypeptide obtenu a la séquence d'acides aminés suivante:

Glu-Cys-Glu-Ser-Gly-Pro-Cys-Cys-Arg-Asn-Cys-Lys-Phe-Leu-Lys-Glu-Gly-Thr-Ile-Cys-Lys-Arg-Ala-Arg-Gly-Asp-Asp-Phe-Asp-Asp-Tyr-Cys-Asn-Gly-Lys-Thr-Cys-Asp-Cys-Pro-Arg-Asn-Pro-His-Lys-Gly-Pro-Ala-Thr

**14.** Procédé selon la revendication 1, dans lequel le polypeptide obtenu a la séquence d'acides aminés suivante:

Glu-Cys-Glu-Ser-Gly-Pro-Cys-Cys-Arg-Asn-Cys-Lys-Phe-Leu-Lys-Glu-Gly-Thr-Ile-Cys-Lys-Asn-Ala-Arg-Gly-Asp-Phe-Leu-Asp-Asp-Tyr-Cys-Asn-Gly-Lys-Thr-Cys-Asp-Cys-Pro-Arg-Asn-Pro-His-Lys-Gly-Pro-Ala-Thr

**15.** Procédé pour la purification d'un polypeptide ayant la séquence suivante:

Glu-Cys-Glu-Ser-Gly-Pro-Cys-Cys-Arg-Asn-Cys-Lys-Phe-Leu-Lys-Glu-Gly-Thr-Ile-Cys-Lys-Arg-Ala-Arg-Gly-Asp-Asp-Met-Asp-Asp-Tyr-Cys-Asn-Gly-Lys-Thr-Cys-Asp-Cys-Pro-Arg-Asn-Pro-His-Lys-Gly-Pro-Ala-Thr

comprenant

a) la dissolution de venin de Echis carinatus lyophilisé dans une solution légèrement basique;
(b) la centrifugation de la solution pour obtenir un surnageant;
(c) le chargement du surnageant sur une colonne et
(d) la détermination des fractions contenant une activité d'inhibition de l'agrégation plaquettaire et la concentration sous vide.

Figure 1

```
    met Glu-Cys-Glu-Ser-Gly-Pro-Cys-Cys-Arg-Asn-Cys-Lys-Phe-Leu-Lys-Glu
                                 (1)
AATTCATG GAA TGC GAA TCA GGT CCA TGC TGT CGT AAC TGC AAG TTC CTT AAG GAA
    GTAC CTT ACG CTT AGT CCA GGT ACG ACA GCA TTG ACG TTC AAG GAA TTC CTT
                                 (2)

-Gly-Thr-Ile-Cys-Lys-Arg-Ala-Arg-Gly-Asp-Asp-Leu-Asp-Asp-Tyr-Cys-Asn-Gly
                                             (3)
GGT ACC ATC TGT AAG CGC GCA CGT GGT GAT GAT CTC GAC GAC TAC TGC AAC GGT
CCA TGG TAG ACA TTC GCG CGT GCA CCA CTA CTA GAG CTG CTG ATG ACG TTG CCA
                                             (4)

-Lys-Thr-Cys-Asp-Cys-Pro-Arg-Asn-Pro-His-Lys-Gly-Pro-Ala-Thr  *
                                         (5)
AAG ACC TGT GAC TGC CCG AGA AAC CCA CAC AAG GGT CCA GCT ACC TAA G
TTC TGG ACA CTG ACG GGC TCT TTG GGT GTG TTC CCA GGT CGA TGG ATT CTTAA
                                         (6)
```

Figure 2

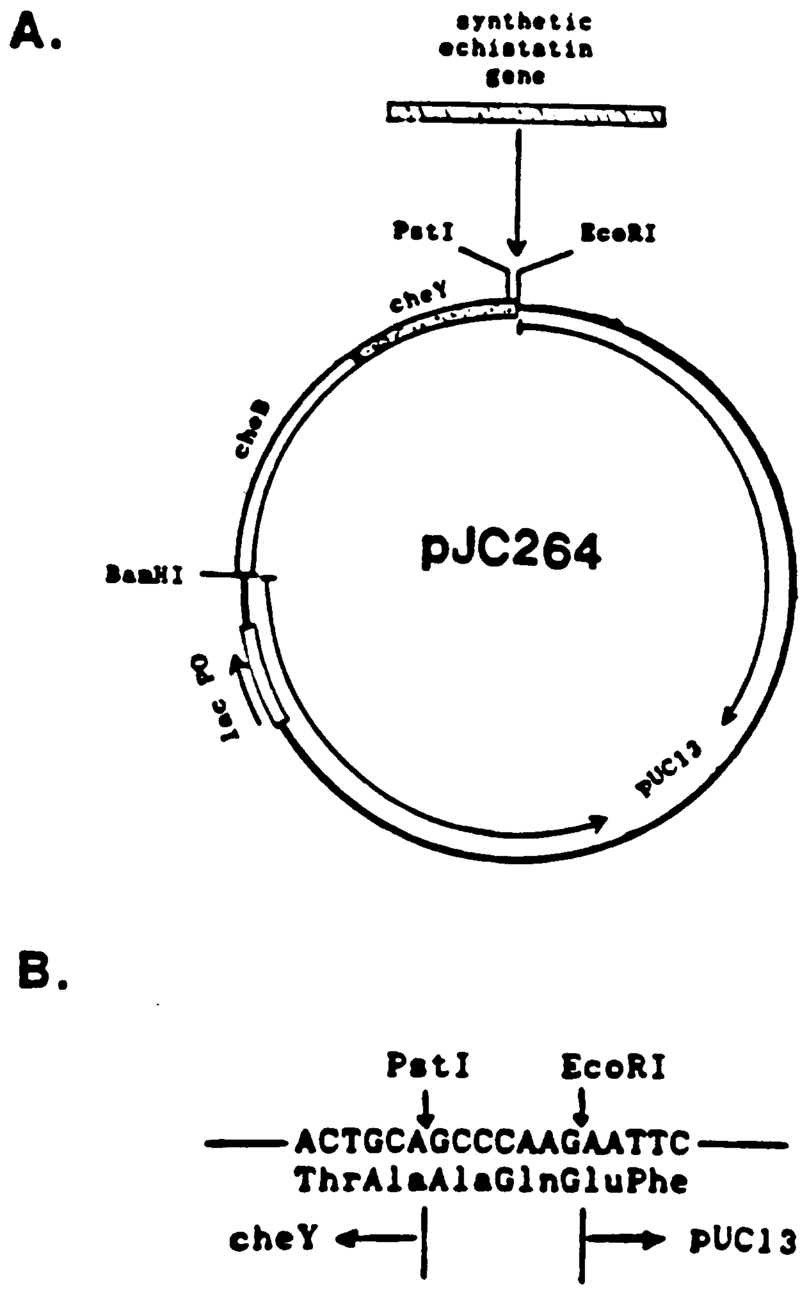
A.
synthetic
echistatin
gene
PstI
EcoRI
cheY
cheB
pJC264
BamHI
lac PO
pUC13
B.
PstI
EcoRI
ACTGCAGCCCAAGAATTC
ThrAlaAlaGlnGluPhe
cheY
pUC13

Figure 3

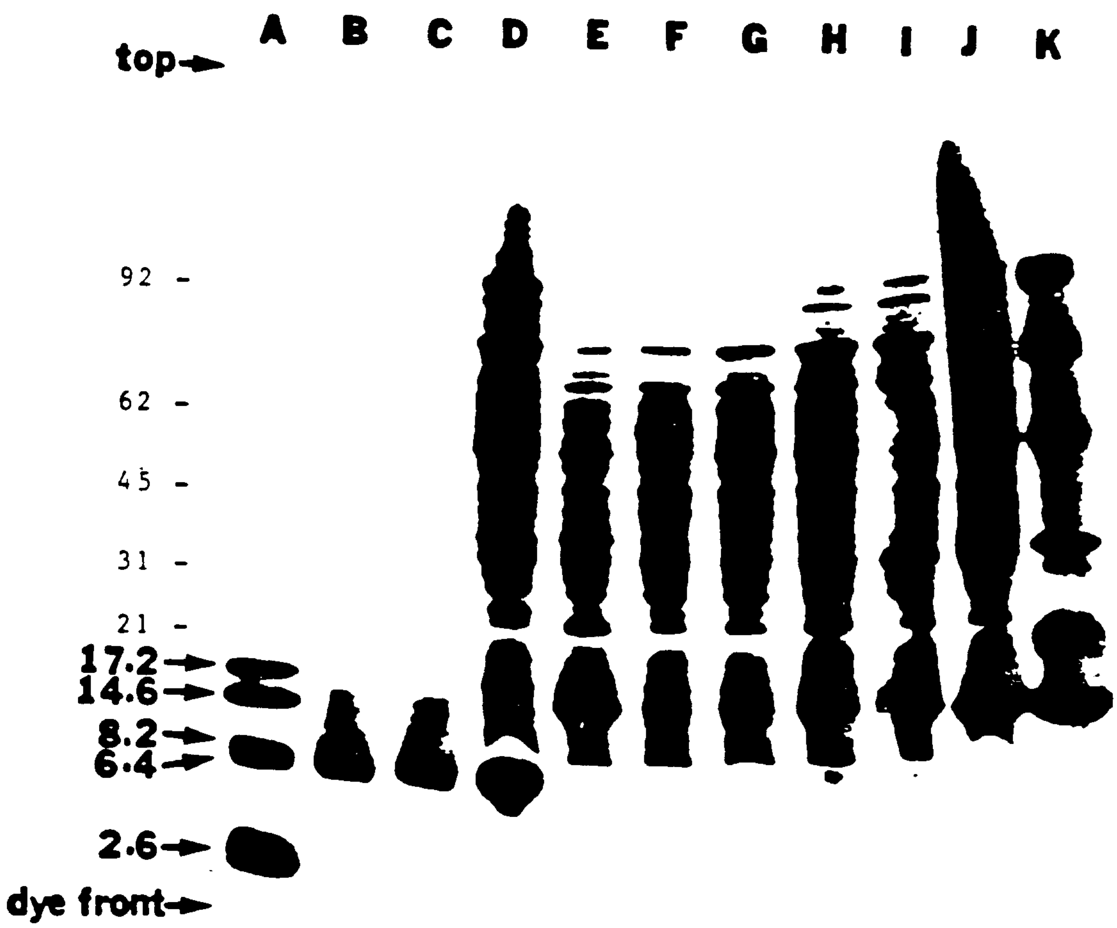

Figure 4

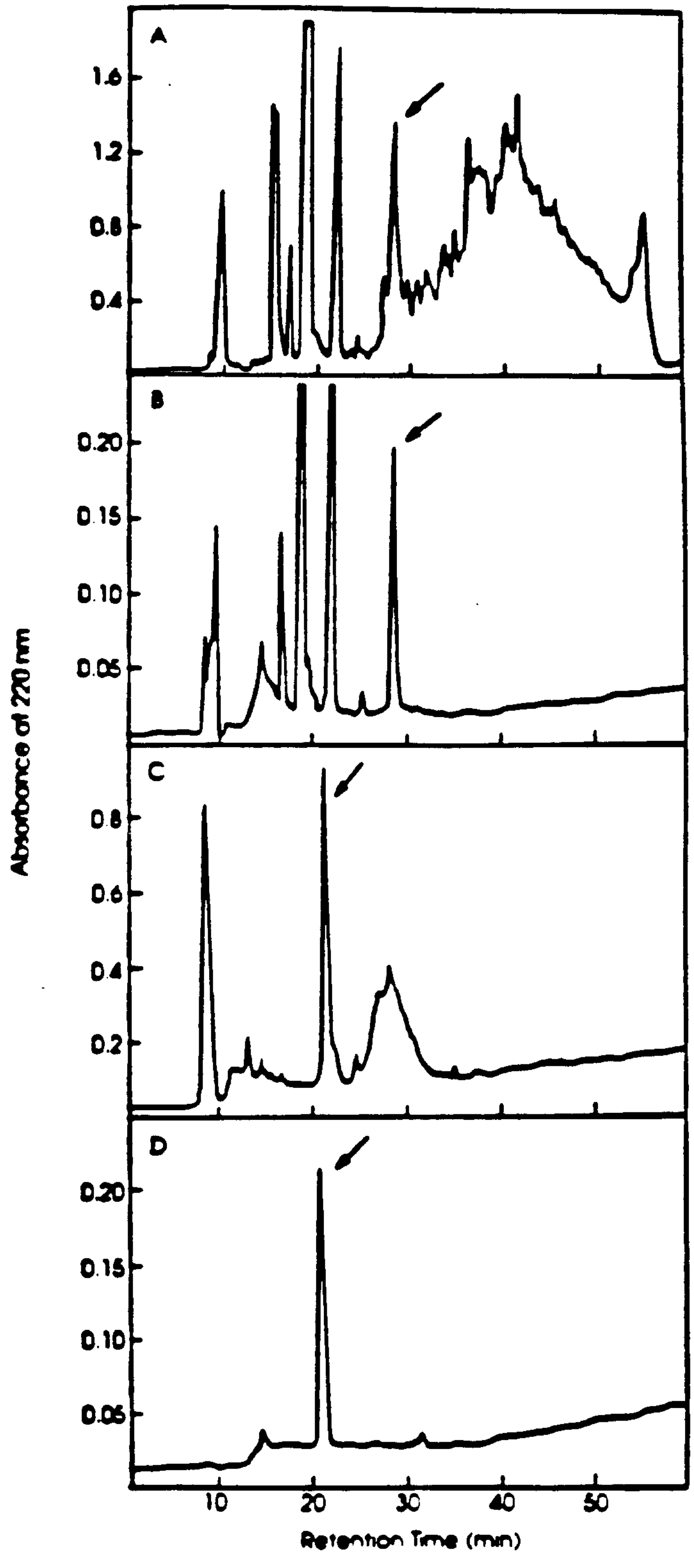
A
B
C
D
1.6
1.2
0.8
0.4
0.20
0.15
0.10
0.05
0.8
0.6
0.4
0.2
0.20
0.15
0.10
0.05
10
20
30
40
50
Absorbance at 220 nm
Retention Time (min)

Figure 5A

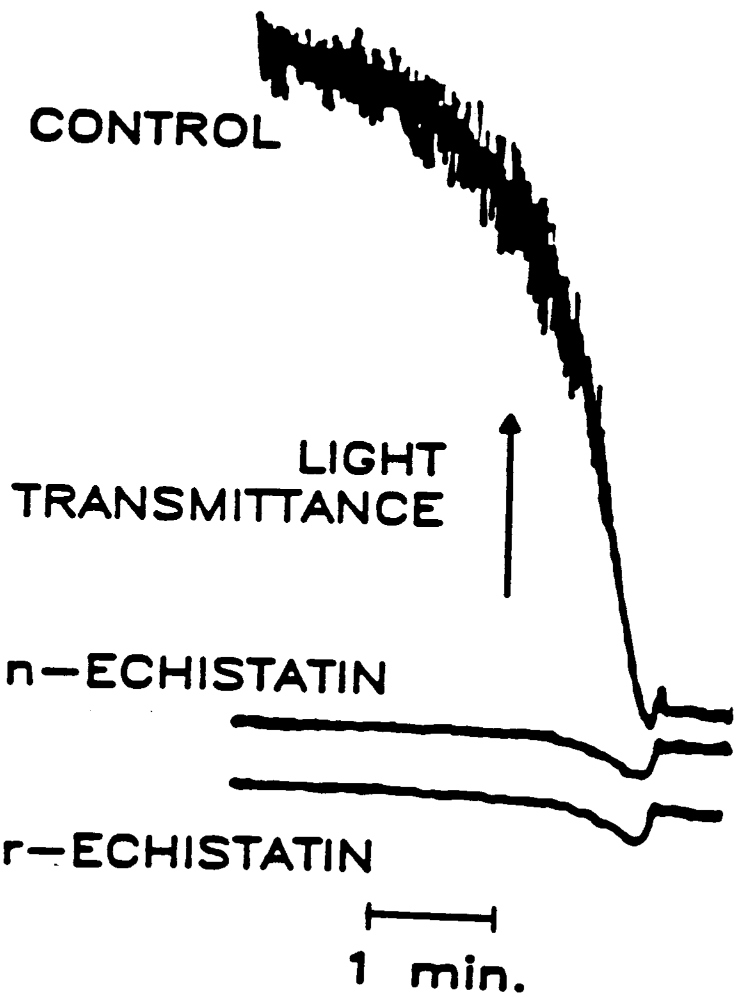
CONTROL
LIGHT
TRANSMITTANCE
n—ECHISTATIN
r—ECHISTATIN
1 min.

Figure 5B

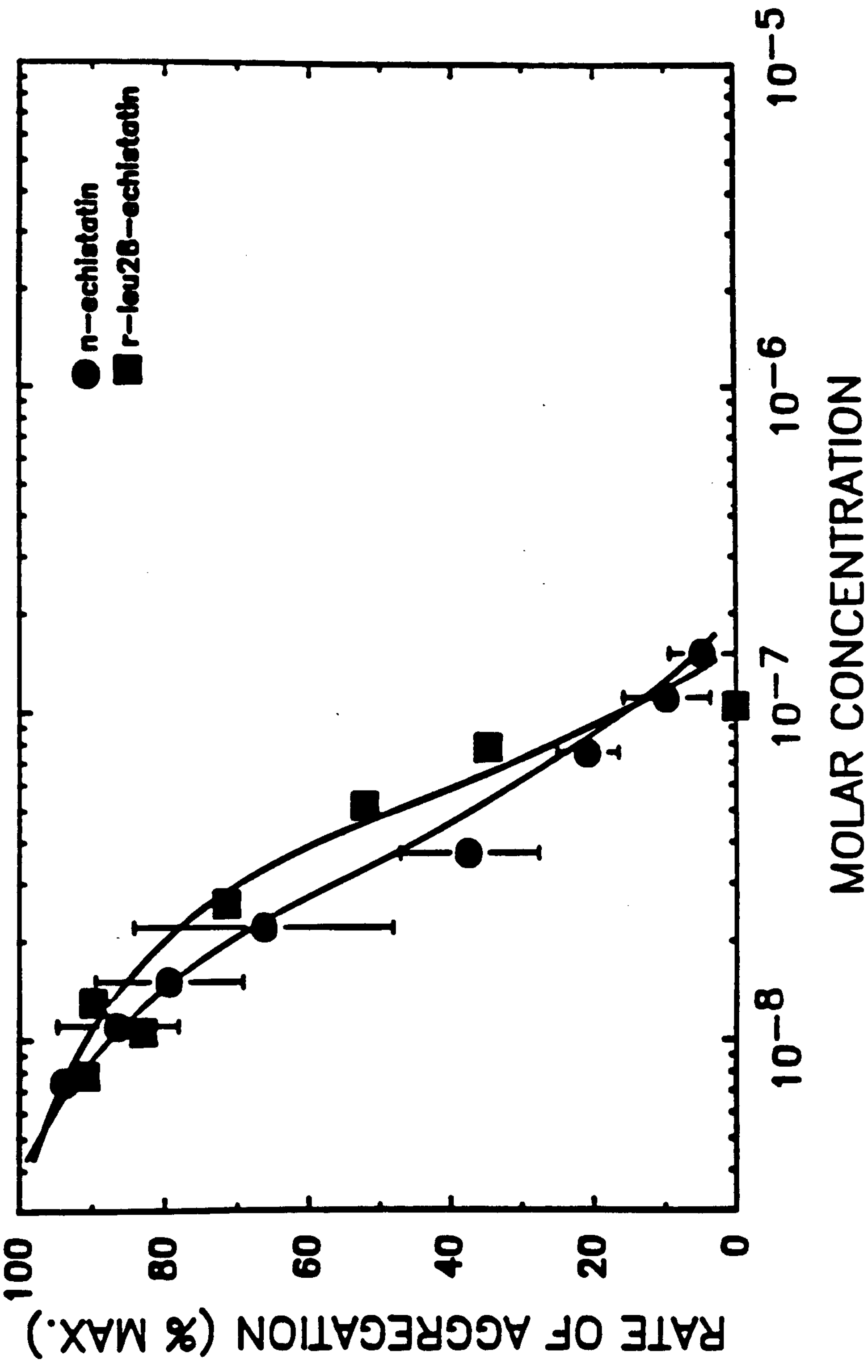
RATE OF AGGREGATION (% MAX.)
100
80
60
40
20
0
MOLAR CONCENTRATION
10−8
10−7
10−6
10−5
n−echistatin
r−leu28−echistatin